# EUROPEAN PATENT APPLICATION

(11) **EP 4 095 145 A1**
(43) Date of publication of application: **30.11.2022**
(21) Application number: 20915832.8
(22) Date of filing: 16.12.2020
(51) Int. Cl.: C07H 15/203, C12N 9/10, C12N 15/09, C12P 19/00

(54) **NEW CARBON GLYCOSIDE GLYCOSYLTRANSFERASE AND USE THEREOF**

(30) Priority: 20.01.2020 CN 202010066924
(71) Applicant: CAS Center for Excellence in Molecular Plant Sciences, Shanghai 200032 (CN)
(72) Inventor: WANG, Yong, Shanghai 200032 (CN); SUN, Yuwei, Shanghai 200032 (CN); CHEN, Zhuo, Shanghai 200032 (CN)
(74) Representative: EP&C
(86) International application number: PCT/CN2020/136886
(87) International publication number: WO 2021/147575

(57) **Abstract**

Provided is a group of new uridine diphosphate (UDP)-glycosyltransferases, which are carbon glycoside glycosyltransferases, wherein the glycosyltransferases can specifically and efficiently catalyze the carbon glycoside glucosylation of a dihydrochalcone(s) compound or a 2-hydroxyflavanone(s) compound, thereby producing a carbon glycoside dihydrochalcone(s) compound or a carbon glycoside-2-hydroxyflavanone(s) compound; and a flavonoid carbon glycoside(s) compound is formed from a carbon glycoside-2-hydroxyflavanone(s) compound by means of a further dehydration reaction. Further provided is the use of said new UDP glycosyltransferases in artificially constructed recombinant expression systems to produce a carbon glycoside dihydrochalcone(s) compound or a flavonoid carbon glycoside(s) compound by means of fermentation engineering.

## Description

### TECHNICAL FIELD

The disclosure belongs to the field of synthetic biology. More specifically, the disclosure relates to a new carboglycoside glycosyltransferase (C-glycosyltransferase) and use thereof.

### BACKGROUND

Flavonoids are a class of polyphenolic hydroxyl compounds with C₆-C₃-C₆ as the parent structure. According to the difference in the parent structure and the substitution of functional groups, they can be divided into flavonoids, flavonols, flavanones, anthocyanins, and chalcone etc. Flavonoids often exist in the form of glycosides in plants, and such glycosylation modifications are mainly catalyzed by uridine diphosphate (UDP)-glycosyltransferase (UGT). The flavonoid glycosides in which the glycosyl and the aglycone parent structure are directly connected by C-C bonds are called C-glycosylated flavones.

C-glycosylated flavone compounds have a variety of pharmacological activities. It is reported that C-glycosylated flavone compounds have significant antioxidant effects, which can significantly inhibit exogenous and endogenous free radicals. Meanwhile, they have certain inhibiting function on bacteria and viruses. C-glycosylated flavone compounds can significantly reduce the content of total cholesterol in the blood, and have activity in the treatment of cardiovascular and cerebrovascular diseases. At the same time, C-glycosylated flavone compounds have significant anti-radiation and neuroprotective effects. Some investigations have shown that C-glycosylated flavone compounds have the effect of anti-metabolic diseases, and have certain therapeutic effects on diseases such as diabetes and obesity.

However, C-glycosylated flavone compounds are rarer than oxyglycoside compounds, and so far only dozens of C-glycosylated flavone have been isolated from nature. In addition, C-glycosylated flavone compounds can only be isolated from nature at present, and large-scale and low-cost production processes such as biosynthesis or artificial synthesis require further research and development.

### SUMMARY OF DESCRIPTION

The purpose of the present disclosure is to provide a new carboglycoside glycosyltransferase (C-glycosyltransferase) and use thereof.

In the first aspect of the present disclosure, provided is a method of catalyzing a dihydrochalcone compound or a 2-hydroxyflavanone compound to generate a carboglycoside dihydrochalcone compound or a carboglycoside-2-hydroxyflavanone compound, comprising: performing the catalysis with a glycosyltransferase; the glycosyltransferase is selected from the polypeptides of any one of SEQ ID NO: 1-20 or a conservative variant thereof.

In a preferred embodiment, the dihydrochalcone compound has a parent structure of formula (I), and the 2-hydroxyflavanone compound (preferably in an open ring form) has a parent structure of formula (II), the carboglycoside dihydrochalcone compound has a parent structure of formula (III), or the carboglycoside-2-hydroxyflavanone compound (preferably in an open ring form) has a parent structure of formula (IV):

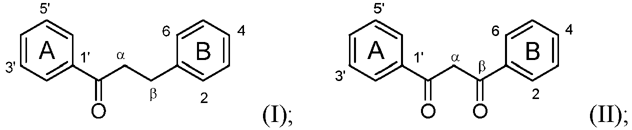

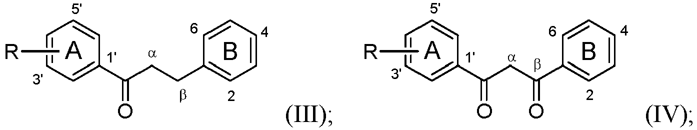

Wherein, R is a glycosyl connected with the A ring by a carbon-carbon bond.

In another preferred embodiment, the A ring or the B ring contains 1 to 3 hydroxyl group(s); preferably, in the A ring, the hydroxyl group is at position 2', 4' and/or 6'; preferably, in the B ring, the hydroxyl group is at position 4.

In another preferred embodiment, the number of R on the A ring is 1, preferably, R is at position 3'.

In another preferred embodiment, the number of R on the A ring is 2, preferably, R is at position 3' and 5'; and the glycosyltransferase is selected from the polypeptide of SEQ ID NO: 6, 8, 10, 17, 19 or 20 or a conservative variant thereof.

In another preferred embodiment, the dihydrochalcone compound or the 2-hydroxyflavanone compound (preferably in an open-ring form) comprises but is not limited to: phloretin, 2-hydroxynaringenin (preferably in an open-ring form), 2-hydroxynaringenin (preferably in an open-ring form), 2-hydroxyeriodictyol.

In another preferred embodiment, the carboglycoside dihydrochalcone compound or carboglycoside-2-hydroxyflavanone compound includes but is not limited to: Nothofagin, phloretin-3',5'-*C*-glucose dicarboglycoside, 2-hydroxynaringenin-6-C(8-C)-glucoside, 2-hydroxyeriodictyol-6-*C*(8-*C*)-glucoside.

In another preferred embodiment, the conservative variant polypeptide is selected from the group consisting of:(1) a polypeptide having one or more (such as 1-20, preferably 1-10, more preferably 1-5, more preferably 1-3) amino acids substituted, deleted, or inserted in the sequence of any of SEQ ID NOs: 1-20, and still having the function of catalyzing a dihydrochalcone compound or a 2-hydroxyflavanone compound to generate a carboglycoside dihydrochalcone compound or a carboglycoside-2-hydroxyflavanone compound; (2) a polypeptide having more than 80% (preferably more than 85%, more preferably more than 90%, more preferably more than 95%, more preferably more than 99%) identity with the amino acid sequence of any of SEQ ID NOs: 1-20, and still having the function of catalyzing a dihydrochalcone compound or a 2-hydroxyflavanone compound to generate a carboglycoside dihydrochalcone compound or a carboglycoside-2-hydroxyflavanone compound; or (3) a polypeptide having a tag sequence at the N- or C-terminus of the polypeptide of any of SEQ ID NOs: 1 to 20, or having a signal peptide at its N-terminus.

In another preferred embodiment, provided is use of a glycosyltransferase in catalyzing a dihydrochalcone compound or a 2-hydroxyflavanone compound to generate a carboglycoside dihydrochalcone compound or a carboglycoside-2-hydroxyflavanone compound; wherein the glycosyltransferase is selected from the polypeptides of any one of SEQ ID NO: 1-20 or a conservative variant thereof.

In a preferred embodiment, the dihydrochalcone compound has a parent structure of formula (I), and the 2-hydroxyflavanone compound (preferably in an open ring form) has a parent structure of formula (II), the carboglycoside dihydrochalcone compound has a parent structure of formula (III), or the carboglycoside-2-hydroxyflavanone compound (preferably in an open ring form) has a parent structure of formula (IV):

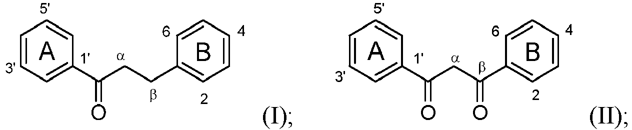

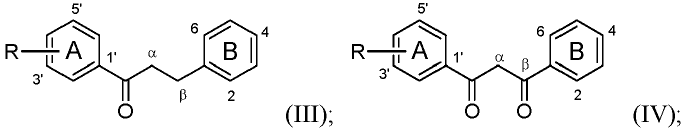

Wherein, R is a glycosyl connected with the A ring by a carbon-carbon bond.

In another preferred embodiment, the A ring or the B ring contains 1 to 3 hydroxyl group(s); preferably, in the A ring, the hydroxyl group is at position 2', 4' and/or 6'; preferably, in the B ring, the hydroxyl group is at position 4.

In another preferred embodiment, the number of R on the A ring is 1, preferably, R is at position 3'.

In another preferred embodiment, the number of R on the A ring is 2, preferably, R is at position 3' and 5'; and the glycosyltransferase is selected from the polypeptide of SEQ ID NO: 6, 8, 10, 17, 19 or 20 or a conservative variant polypeptide thereof.

In another preferred embodiment, R is a mono-glycosyl.

In another preferred embodiment, the glycosyl is a glucose; preferably, when the glycosyltransferase is used for glycosyl transfer, a compound carrying a glucose group is used as a donor; more preferably, UDP glucose is used as the glycosyl donor.

In another preferred embodiment, the dihydrochalcone compound or the 2-hydroxyflavanone compound comprises but is not limited to: phloretin, 2-hydroxynaringenin (preferably in an open-ring form), 2-hydroxyeriodictyol (preferably in an open-ring form).

In another preferred embodiment, the carboglycoside dihydrochalcone compound or carboglycoside-2-hydroxyflavanone compound includes but is not limited to: Nothofagin, phloretin-3',5'-*C*-glucose carboglycoside, 2-hydroxynaringenin-6-C(8-C)-glucoside, 2-hydroxyeriodictyol-6-*C*(8-*C*)-glucoside.

In another aspect of the present disclosure, provided is a method of synthesizing (including artificial synthesizing or *in vitro* synthesizing) a C-glycosylated flavone compound, comprising: (1) catalyzing 2-hydroxyflavanone compound by a glycosyltransferase to produce carboglycoside-2-hydroxyflavanone compound; wherein, the glycosyltransferase is selected from the polypeptides of any one of SEQ ID NO: 1-20 or a conservative variant thereof; (2) dehydrating the carboglycoside-2-hydroxyflavanone compound of (1) to obtain a C-glycosylated flavone compound.

In another preferred embodiment, the method further comprises step (c) before (1): (c) catalyzing the flavanone compound by flavanone-2-hydroxylase (F2H) to obtain 2-hydroxyflavanone compound.

In another preferred embodiment, the method further comprises step (b) before (c): (b) catalyzing malonyl-CoA structural analogs (such as including the substitution form with one or more groups substituted) and *p*-coumaroyl-CoA structural analogs (such as including the substitution form with one or more groups substituted) by chalcone synthetase (CHS) and chalcone isomerase (CHI) to obtain flavanone compounds.

In another preferred embodiment, the method further comprises step (a) before (b): (a) catalyzing aromatic amino acids by tyrosine ammonia lyases (TAL) or phenylalanine ammonia lyase (PAL) and 4-coumaroyl-CoA ligase (4CL), to obtain *p*-coumaroyl-CoA structural analogs.

In another preferred embodiment, the flavanone compounds comprise: naringenin, eriodictyol; the malonyl-CoA structural analogs include: malonyl-CoA or methylmalonyl-CoA; the *p*-coumaroyl-CoA structural analogs include: *p*-coumaroyl-CoA or *p*-cinnamoyl-CoA; the aromatic amino acids include: L-tyrosine or L-phenylalanine.

In another preferred embodiment, the 2-hydroxyflavanone compound is 2-hydroxynaringenin, which is obtained from naringenin catalyzed by flavanone-2-hydroxylase (F2H); or the 2-hydroxyflavanone compound is 2-hydroxyeriodictyol, which is obtained from eriodictyol catalyzed by flavanone-2-hydroxylase (F2H); preferably, the eriodictyol is obtained from naringenin catalyzed by flavanone-3'-hydroxylase (F3'H).

In another aspect of the present disclosure, provided is a method of biosynthesizing a C-glycosylated flavone compound, comprising: (i) co-transforming into a host cell precursor gene(s) for the synthesis of naringenin compound, the gene encoding flavanone-2-hydroxylase and/or flavanone-3'-hydroxylase, and the gene encoding glycosyltransferase; wherein, the glycosyltransferase is selected from the polypeptides of any one of SEQ ID NO: 1-20 or a conservative variant thereof; (ii) culturing the cells of (i) to biosynthesize C-glycosylated flavone compounds.

In another preferred embodiment, the flavanone-2-hydroxylase and flavanone-3'-hydroxylase are P450 oxidase with the N-terminal transmembrane region truncated; preferably, P450 oxidase is added with a tag after truncating the N-terminal transmembrane region; more preferably, the tag includes but is not limited to: 2B1, 17α, MBP.

In another aspect of the present disclosure, provided is a genetically engineered cell, comprising precursor gene(s) for the synthesis of naringenin compound, the gene encoding flavanone-2-hydroxylase and/or flavanone-3'-hydroxylase, and the gene encoding glycosyltransferase; wherein, the glycosyltransferase is selected from the polypeptides of any one of SEQ ID NO: 1-20 or a conservative variant thereof.

In another aspect of the present disclosure, provided is a method for preparing the cell of claim 21, comprising: co-transforming into a host cell precursor gene(s) for the synthesis of naringenin compound, the gene encoding flavanone-2-hydroxylase and/or flavanone-3'-hydroxylase, and the gene encoding glycosyltransferase; wherein, the glycosyltransferase is selected from the polypeptides of any one of SEQ ID NO: 1-20 or a conservative variant thereof.

In another aspect of the present disclosure, provided is a kit for biosynthesizing a C-glycosylated flavone compound or its intermediate, comprising: one or more (such as 2, 3, 4) polypeptides shown in SEQ ID NOs: 1-20 or conservative variant polypeptides thereof; flavanone-2-hydroxylase and/or flavanone-3'-hydroxylase; precursor gene(s) for synthesizing naringenin compounds; preferably, the kit also comprising host cells.

In another preferred embodiment, the cells include: prokaryotic cells or eukaryotic cells; preferably, the prokaryotic host cells include *Escherichia coli,* yeast, streptomyces.

In another preferred embodiment, the C-glycosylated flavone compound comprises: vitexin, isovitexin, orientin, isoorientin, vitzenin-2 (New Zealand vitexin), lucenin (lucenin 2).

Other aspects of the disclosure will be apparent to those skilled in the art based on the disclosure herein.

### BRIEF DESCRIPTION OF THE DRAWINGS

Figure 1. *In vitro* functional identification of C-glycosyltransferase by HPLC; (A) using phloretin as substrate; (B) using 2-hydroxynaringenin as substrate.
Figure 2. High-resolution secondary MS spectrum in negative ion mode of Nothofagin.
Figure 3. (A)¹H, (B)¹³C and (C) two-dimensional HMBC NMR spectra of Nothofagin.
Figure 4. The secondary MS spectrum of 2-hydroxynaringenin-C-glucoside.
Figure 5. Mie equation curves of PhCGT1 and PgCGT1 for the substrate 2-hydroxynaringenin.
Figure 6. The result of high-resolution mass spectrometry detection in negative ion mode of dihydrochalcone di-carboglycoside (phloretin-3',5'-*C*-diglucoside).
Figure 7. (A) Schematic diagram of pYH055 synthetic naringenin gene expression cassette; (B) schematic diagram of pCZ201, pCZ203 and pCZ229 carrying P450/CPR expression cassette.
Figure 8. Schematic diagram of pCZ261 and pCZ265 carrying a binary P450/CPR expression cassette.
Figure 9. LC-MS analysis results of the fermentation broths of vitexin and isovitexin production strains sCZ2, sCZ4 and sCZ29.
Figure 10. LC-MS analysis results of the fermentation broths of orientin and isoorientin production strains sCZ63 and sCZ67.
Figure 11. Prospective biosynthetic pathway of flavonoid-monocarboglycoside.
Figure 12. Prospective biosynthetic pathway of vitzenin-2 (apigenin-6,8-diglucoside).
Figure 13. HPLC and LC-MS detection of the components of the fermentation product of vitzenin-2 production strain.

### DETAILED DESCRIPTION

After in-depth research, the present inventors screened out a group of novel uridine diphosphate(UDP)-glycosyltransferases, which are C-glycosyltransferases. The glycosyltransferase can specifically and efficiently catalyze the carboglycoside glucosylation of dihydrochalcone compounds or 2-hydroxyflavanone compounds, thereby producing a type of carboglycoside dihydrochalcone compound or carboglycoside-2-hydroxyflavanone compound; carboglycoside-2-hydroxyflavanone compound forms C-glycosylated flavone compound through further dehydration reaction. The present disclosure also relates to applying these novel UDP glycosyltransferases to artificial recombinant expression systems to produce carboglycoside dihydrochalcone compounds or C-glycosylated flavone compounds through fermentation engineering.

### Active polypeptide, encoding gene thereof, vector and host

The present inventors have explored genomic information and transcriptome information and conducted a lot of research and experimental work, and revealed a new group of uridine diphosphate (UDP)-glycosyltransferases, which are C-glycosyltransferase (*C*-glycosyltransferase, CGT). Preferably, the group of C-glycosyltransferases are derived from monocotyledonous gramineae; more preferably, derived from moso bamboo (*Phyllostachys edulis* or *Phyllostachys heterocycle; Ph),* henon bamboo *(Phyllostachys glauca; Pg),* High knot bamboo *(Phyllostachys prominens; Pp),* Japonica (*Oryza Sativa* japonica; *Os*)*,* maize (*Zea mays; Zm*)*,* wheat (*Triticum aestivum; Ta*)*,* Sorghum (*Sorghum bicolor; Sb*)*, Brachypodium distachyo* (*Bd*)*,* millet (*Setaria italica; Si).*

As a preferred embodiment of the present disclosure, the C-glycosyltransferase comprises a glycosyltransferase selected from the group consisting of: PhCGT1 (SEQ ID NO: 1), PgCGT1 (SEQ ID NO: 2), PpCGT1 (SEQ ID NO: 3), PhCGT2(SEQ ID NO: 4), OsCGT2(SEQ ID NO: 5), SbCGT1 (SEQ ID NO: 6), SbCGT2(SEQ ID NO: 7), TaCGT1-A(SEQ ID NO: 8), TaCGT1-B(SEQ ID NO: 9), TaCGT1-D (SEQ ID NO: 10), TaCGT2-A(SEQ ID NO: 11), TaCGT2-B(SEQ ID NO: 12), TaCGT2-D (SEQ ID NO: 13), ZmCGT4(SEQ ID NO: 14), ZmCGT5(SEQ ID NO: 15), BdCGT1 (SEQ ID NO: 16), SiCGT1(SEQ ID NO: 17), SiCGT2(SEQ ID NO: 18), OsCGT(SEQ ID NO: 19), ZmCGT(SEQ ID NO: 20).

The present disclosure also includes conservative variant polypeptides of the C-glycosyltransferase, and the present disclosure also includes conservative variant polypeptides of flavanone-2-hydroxylase, chalcone synthase, chalcone isomerase, tyrosine ammonia lyase, phenylalanine ammonia lyase, 4-coumaroyl-CoA ligase, etc. In the present disclosure, the "conservative variant polypeptide" refers to a polypeptide that basically maintains the same biological function or activity of the polypeptide. The "conservative variant polypeptide" may be (i) polypeptides with one or more conservative or non-conservative amino acid substitution (preferably conservative), where the substituted amino acid residues may or may not be one encoded by the genetic code, or (ii) polypeptides containing substitutions of one or more amino acid residues having a substituent group, or (iii) polypeptides formed having the mature polypeptide fused with another compound (such as compounds that extend half-life of the polypeptide, for example, polyethylene glycol), or (iv) polypeptides formed by having said polypeptide fused with additional amino acid sequence (such as leader sequence or secretory sequence, or sequence used for purification of the polypeptideor proprotein sequence, or fusion protein formed with fragment of antigen IgG). In accordance with the teachings provided herein, these fragments, derivatives and analogs are well known to a person skilled in the art.

The "conservative variant polypeptide" may include but are not limited to: deletion, insertion and/or substitution of one or more (usually 1-50, preferably 1-30, more preferably 1-20, most preferably 1-10) amino acids, and addition or deletion of one or several (usually within 50, preferably within 20 or 10, more preferably within 5) amino acids at the C-terminal and/or N-terminal. For example, substitution with amino acids of comparable or similar properties usually does not change protein function in the art. As another example, addition of one or more amino acids to the C-terminus and/or N-terminus usually does not change the function of a protein either. The present disclosure also provides analogs of the polypeptides. The differences between analogs and the original polypeptide may be the difference in amino acid sequences, and may also be the difference in the forms of modifications that will not affect the sequence, or both. These polypeptides include natural or induced genetic variants. Induced variants can be obtained by a variety of techniques, such as generating random mutagenesis by irradiation or exposure to mutagens, and can also be obtained by directed mutagenesis or other known molecular biology techniques. Analogs mentioned herein also include analogs with residue(s) different from natural L-amino acid (e.g., D-amino acids), as well as analogs with a non-naturally occurred or synthetic amino acid (such as β, γ-amino acids). It should be understood that the polypeptides of the present disclosure are not limited to the representative polypeptides described above.

As a preferred embodiment of the present disclosure, the P450 oxidase (flavanone-2-hydroxylase and flavanone-3'-hydroxylase) described in the present disclosure is a polypeptide fragment with the N-terminal transmembrane region removed, which can result in a truncated protein having increased soluble expression and exert better activity when used in biosynthetic pathways.

Therefore, the N-terminus or C-terminus of the C-glycosyltransferase herein may further contain one or more polypeptide fragments as protein labels. For example, the labels may be FLAG, HA, HA1, c-Myc, Poly-His, Poly-Arg, Strep-TagII, AU1, EE, T7, 4A6, ε, B, gE, and Ty1. For another example, the labels may include 2B, 17α, MBP and the like.

For the purpose of producing the C-glycosyltransferase of the present disclosure, a signal peptide sequence can also be added to the amino terminus of the polypeptide of the present disclosure so that to secrete the expressed protein (e.g., to be secreted outside the cell). The signal peptide can be cleaved during secretion of the polypeptide from the cell.

The active polypeptides of the present disclosure can be recombinant polypeptides, natural polypeptides, and synthetic polypeptides. The polypeptide of the disclosure can be a naturally purified product, or a chemically synthesized product, or can be produced by prokaryotic or eukaryotic hosts (such as bacteria, yeast, higher plants) using recombination technology. Depending on the host used in the recombinant production, the polypeptide of the disclosure may be glycosylated or non-glycosylated. Polypeptides of the disclosure may or may not include an initial methionine residue.

The polynucleotide sequences encoding the C-glycosyltransferase of the present disclosure can be in the form of DNA or RNA. Forms of DNA include cDNA, genomic DNA or artificially synthesized DNA. DNA can be single-stranded or double-stranded. The DNA may be coding strand or non-coding strand. The term "polynucleotide encoding a/the polypeptide" can include a polynucleotide encoding the polypeptide, or a polynucleotide that further includes additional coding and/or non-coding sequences.

The disclosure also relates to vectors comprising the polynucleotide of the disclosure, as well as host cells genetically engineered using the vectors or coding sequences of the polypeptide disclosed herein, and a method for producing the polypeptides of the disclosure by recombination technology.

Recombinant polypeptides can be expressed or produced by conventional recombinant DNA techniques. Generally speaking, there are the following steps: (1) transforming or transducing an appropriate host cell with a polynucleotide encoding the polypeptide (including a conservative variant polypeptide thereof) or with a recombinant expression vector containing the polynucleotide; (2) culturing the host cells in a suitable medium; (3) isolating and purifying proteins from culture media or cells.

In the disclosure, the sequence of the polynucleotide encoding the polypeptide can be inserted into a recombinant expression vector. The term "recombinant expression vector" refers to bacterial plasmid, phage, yeast plasmid, plant cell virus, mammalian cell virus (such as adenoviruses, retroviruses) or other vectors well known in the art. Any plasmid or vector can be used, provided that it can replicate and be stable in the host. An important charecterastic of an expression vector is that it usually contains an origin of replication, a promoter, a marker gene and a translation control element. Preferably, the expression vector may be a prokaryotic expression vector.

Suitable methods for constructing expression vector which comprises the polynucleotide encoding the C-glycosyltransferase of the disclosure and appropriate transcriptional/translational control signals are well known to the person skilled in the art. These methods include *in vitro* recombinant DNA technology, DNA synthesis technology, *in vivo* recombinant technology and so on. Said DNA sequence may be effectively linked to a proper promoter in the expression vector to direct mRNA synthesis. In addition, the expression vector preferably contains one or more selective marker genes to provide phenotypic traits for the selection of transformed host cells.

Vectors containing the above appropriate DNA sequences and appropriate promoters or regulatory sequences can be used to transform appropriate host cells so that they can express proteins. The host cell can be a prokaryotic cell, such as a bacterial cell; or a lower eukaryotic cell, such as a yeast cell; or a higher eukaryotic cell, such as a mammalian cell. Representative examples are: bacterial cells such as cells of *Escherichia coli, Streptomyces; Bacillus subtilis, Salmonella typhimurium;* fungal cells such as yeast cells, plant cells, Ganoderma lucidum cells; insect cells such as *Drosophila* S2 or Sf9 cells; animal cells such as CHO, COS, 293 cells, or Bowes melanoma cells, etc.

The present disclosure also provides host cells for biosynthesizing C-glycosylated flavone compounds or intermediates thereof, including: precursor genes for synthesizing naringenin compounds, genes encoding flavanone-2-hydroxylase and/or flavanone-3'-hydroxylase and the gene encoding glycosyltransferase of the present disclosure.

In a preferred embodiment of the present disclosure, the host cells are prokaryotic cells, more preferably *Escherichia coli,* yeast, and *Streptomyces.* It should be understood that the cell host is only a production tool, and those skilled in the art can modify host cells other than *Escherichia coli* by technical means, and realize the biosynthesis of the present disclosure. The host cells thus constituted and the production method should also be included in the present disclosure.

### Use and production process

The C-glycosyltransferase or a conservative variant polypeptide thereof can specifically and efficiently catalyze the carboglycoside glucosylation of dihydrochalcone compounds or 2-hydroxyflavanone compounds, thereby producing a type of carboglycoside dihydrochalcone compound or carboglycoside-2-hydroxyflavanone compound; carboglycoside-2-hydroxyflavanone compound forms C-glycosylated flavone compound through further dehydration reaction.

Therefore, the disclosure provides use of a glycosyltransferase in catalyzing a dihydrochalcone compound or a 2-hydroxyflavanone compound to generate a carboglycoside dihydrochalcone compound or a carboglycoside-2-hydroxyflavanone compound; wherein the glycosyltransferase is selected from the polypeptides of any one of SEQ ID NO: 1-20 or a conservative variant thereof.

As used in the present disclosure, the dihydrochalcone compounds include dihydrochalcone or its derivatives, structural analogs, and isomers. The 2-hydroxyflavanone compounds include 2-hydroxyflavanone or its derivatives, structural analogs and isomers. The carboglycoside dihydrochalcone compounds include carboglycoside dihydrochalcone or its derivatives, structural analogs, and isomers. The carboglycoside-2-hydroxyflavanone compounds include carboglycoside-2-hydroxyflavanone or its derivatives, structural analogs and isomers. The C-glycosylated flavones compounds include C-glycosylated flavones or derivatives, structural analogs and isomers thereof.

In a preferred embodiment of the disclosure, the dihydrochalcone compound has a parent structure of formula, and the 2-hydroxyflavanone compound (open ring form) has a parent structure of formula (II), the carboglycoside dihydrochalcone compound has a parent structure of formula (III), or the carboglycoside-2-hydroxyflavanone compound (open ring form) has a parent structure of formula (IV):

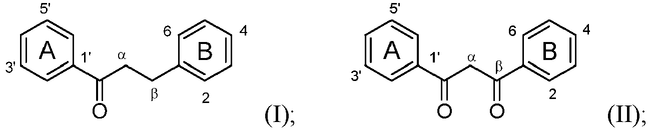

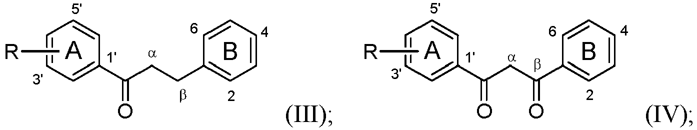

Wherein, R is a glycosyl connected with the A ring by a carbon-carbon bond. The glycosyl can be a mono-glycosyl, a diglycosyl or a polyglycosyl (e.g., 3 or more (eg, 3-10, more specifically, 4, 5, 6, 7, 8, 10) monosaccharide in series). In a preferred embodiment, the glycosyl is a monoglycosyl.

In another preferred embodiment of the disclosure, the A ring or the B ring contains 1 to 3 hydroxyl group(s); preferably, in the A ring, the hydroxyls is at position 2', 4' and/or 6'; preferably, in the B ring, the hydroxyls is at position 4.

In another preferred embodiment of the disclosure, the number of R on the A ring is 1, preferably, R is at position 3'; or the number of R on the A ring is 2, preferably, preferably R is at position 3' and 5'; and the glycosyltransferase is selected from the polypeptide of SEQ ID NO: 6, 8, 10, 17, 19 or 20 or a conservative variant polypeptide thereof.

In another preferred embodiment of the disclosure, the dihydrochalcone compound or 2-hydroxyflavanone compound includes: phloretin, 2-hydroxynaringenin (open-ring form), 2-hydroxynaringenin (open-ring form), 2-hydroxyeriodictyol; and/or the carboglycoside dihydrochalcone compound or carboglycoside-2-hydroxyflavanone compound includes: Nothofagin, phloretin-3',5'-*C*-glucose carboglycoside, 2-hydroxynaringenin-6-C(8-C)-glucoside, 2-hydroxyeriodictyol-6-C(8-C)-glucoside. In addition, their analogs or group-substituted variants also should be included.

In the present disclosure, UDP glucose is preferably used as the glycosyl donor, and it should be understood that other compounds carrying a glucose group can also be used as the donor, and should also be included in the present disclosure.

The present disclosure also provides a method of catalyzing a dihydrochalcone compound or a 2-hydroxyflavanone compound to generate a carboglycoside dihydrochalcone compound or a carboglycoside-2-hydroxyflavanone compound, comprising: performing the catalysis with a glycosyltransferase; the glycosyltransferase is selected from the polypeptides of any one of SEQ ID NO: 1-20 or a conservative variant thereof.

The present disclosure also provides a method of synthesizing a C-glycosylated flavone compound, comprising: (1) catalyzing 2-hydroxyflavanone compound by a glycosyltransferase to produce carboglycoside-2-hydroxyflavanone compound; wherein, the glycosyltransferase is selected from the polypeptides of any one of SEQ ID NO: 1-20 or a conservative variant thereof;(2) dehydrating the carboglycoside-2-hydroxyflavanone compound of (1) to obtain a C-glycosylated flavone compound. Preferably, the method further comprises step (c) before (1): (c) catalyzing the flavanone compound by flavanone-2-hydroxylase (F2H) to obtain 2-hydroxyflavanone compound. More preferrably, the method further comprises step (b) before (c): (b) catalyzing malonyl-CoA type compounds (such as including the substitution form with one or more groups substituted) and *p*-coumaroyl-CoA type compounds (such as including the substitution form with one or more groups substituted) by chalcone synthetase (CHS) and chalcone isomerase (CHI) to obtain flavonoid compounds. Further preferably, the method comprises step (a) before (b): (a) catalyzing L-tyrosine type compounds by tyrosine ammonia lyases (TAL) or 4-coumaroyl-CoA ligase (4CL), to obtain p-coumaroyl-CoA type compounds.

In preferred embodiment of the disclosure, the flavonoid compounds comprise: naringenin, eriodictyol; the malonyl-CoA type compounds include: malonyl-CoA, methylmalonyl-CoA; the *p*-coumaroyl-CoA type compounds include: *p*-coumaroyl-CoA, *p*-cinnamoyl-CoA; the L-tyrosine type compounds include: L-tyrosine or L-phenylalanine. It should be understood that, in light of the general disclosure of the present disclosure, their analogs or variants may also be employed in the present disclosure.

In a preferred embodiment of the disclosure, the 2-hydroxyflavanone compound is 2-hydroxynaringenin, which is obtained from naringenin catalyzed by flavanone-2-hydroxylase (F2H); or the 2-hydroxyflavanone compound is 2-hydroxyeriodictyol, which is obtained from eriodictyol catalyzed by flavanone-2-hydroxylase (F2H); preferably, the eriodictyol is obtained from naringenin catalyzed by flavanone-3'-hydroxylase (F3'H). It should be understood that, in light of the general description of the present disclosure, their analogs or variants may also be employed in the present disclosure.

The present disclosure also provides a method of biosynthesizing a C-glycosylated flavone compound, comprising: (i) co-transforming into a host cell precursor gene(s) for the synthesis of naringenin compound, the gene encoding flavanone-2-hydroxylase and/or flavanone-3'-hydroxylase, and the gene encoding glycosyltransferase; wherein, the glycosyltransferase is selected from the polypeptides of any one of SEQ ID NO: 1-20 or a conservative variant thereof; (ii) culturing the cells of (i) to biosynthesize C-glycosylated flavone compounds.

In a specific embodiment of the present disclosure, the fusion proteins and their derived polypeptides obtained by linking the N-terminus or/and C-terminus of these C-glycosyltransferase with tags can specifically and efficiently catalyze *C*-glucosylation of position 3' of 2-hydroxyflavanone compounds or dihydrochalcone compounds, wherein, PhCGT1 has better activity than previously reported similar C-glycosyltransferase, while SbCGT1, TaCGT1-A, TaCGT1-D, SiCGT1, OsCGT and ZmCGT can not only catalyze the 3' *C*-glycosylation of chalcone compounds, but also can further catalyze 5' *C*-glycosylation of the 3'-glycosylated dihydrochalcone compounds or 2-hydroxyflavanone compounds, resulting in the bi-carboglycoside dihydrochalcone compounds or bi-carboglycoside-2-hydroxyflavanone. Mono-carboglycoside or bi-carboglycoside-2-hydroxyflavanone compounds can generate mono-carboglycoside or bi-carboglycoside compounds through non-enzymatic dehydration condensation reaction. On the other hand, the present disclosure applies these novel CGTs to an artificial recombinant *Escherichia coli* system through bioengineering technology, and the recombinant microorganism can produce carboglycoside dihydrochalcone and C-glycosylated flavone compounds.

Compared with traditional plant extraction methods, microbial fermentation has the advantages of fast speed and less influence by external factors. Meanwhile, the yield of some compounds through microbial synthesis is much higher than that of plant extraction. Microbial fermentation has become an important means of obtaining natural products. The natural abundance of C-glycosylated flavone compounds is low, and they coexist with a large number of structurally similar flavonoid oxyglycosides and phenylpropanoids in plant extracts, which makes the separation and purification cumbersome and complicated. In the present disclosure, the method of microbial fermentation is used to efficiently and directionally synthesize C-glycosylated flavones, and the cost of separation and purification of such compounds is extremely effectively reduced.

The present disclosure also provides a kit for biosynthesizing a C-glycosylated flavone compound or its intermediate, comprising: one or more polypeptides shown in SEQ ID NOs: 1-20 or conservative variant polypeptides thereof; flavanone-2-hydroxylase and/or flavanone-3'-hydroxylase; precursor gene(s) for synthesizing naringenin compounds; preferably, the kit also comprising host cells. More preferably, the kit also includes an instruction describing the method of the biosynthesis.

The disclosure is further illustrated by the specific examples described below. It should be understood that these examples are merely illustrative, and do not limit the scope of the present disclosure. The experimental methods without specifying the specific conditions in the following examples generally used the conventional conditions, such as those described in J. Sambrook, Molecular Cloning: A Laboratory Manual (3rd ed. Science Press, 2002) or followed the manufacturer's recommendation.

### Reagent and material

Moso bamboo, henon bamboo, and high knot bamboo were collected from Shanghai Chenshan Botanical Garden.

Japonica rice, sorghum, maize, wheat, millet, and *Brachypodium distachyo* were planted in the greenhouse of Shanghai Molecular Plant Research Center, Chinese Academy of Sciences.

Plant genomic DNA was extracted using TIANGEN Plant Genome Extraction Kit.

AxyPrep total RNA miniprep kit, polymerase chain reaction (PCR) gel recovery kit, and plasmid extraction kit are all products of Axygen (American).

PCR high-fidelity polymerase PrimeSTAR Max DNA Polymerase was purchased from Takara (Japan).

Restriction endonuclease and T4 ligase were purchased from NEB.

Seamless cloning kit was purchased from Vazyme Biotechnology Co., Ltd.

*Escherichia coli* DH10B, Rosetta (DE3) strain and pET21a, pET28a vectors were used for gene cloning and protein expression.

The standard compounds naringenin, 2-hydroxynaringenin, phloretin, orientin, isoorientin, vitexin, and isovitexin were purchased from Dalian Meilun Biotechnology Co., Ltd.

UDP glucose was purchased from Beijing Zhongtai biological Co., Ltd.

Other reagents are analytical grade reagent or chromatographic grade reagent, purchased from Sinopharm Chemical Reagent Co., Ltd.

### Example 1. Novel uridine diphosphate (UDP)-glycosyltransferase

### 1.Cloning of C-glycosyltransferase gene and construction of expression plasmid

Take mature leaves or seedlings of gramineous plants (japonica rice, maize, wheat, sorghum, millet, *Brachypodium distachyo,* Moso bamboo, henon bamboo, high knot bamboo), clean the surface with clean water, and gently wipe dry the surface of the leaves with absorbent paper, weighed for use. The extraction of genomic DNA was performed according to the instructions provided by the TIANGEN Plant Genome Extraction Kit.

The amino acid sequence of the C-glycosyltransferase is as follows:
> PhCGT1 (SEQ ID NO: 1) 
MPTSGGRTGDRPHVILLPSAGMGHLVPFSRLAVALSSGHGCDVSVVAVLPTVSAAESRHLQGLLDASPAVRRLDL
HLAPFDESEFPGADPFFLRFEAMRRSAPLLGPLLADTGASALVTDIALASVVIPVAKEIHLPCYVLFTASAAMLSFCAYFPT
YLDTNAGRVVGDVDIPGVYRIPKASIPQALHDPNHLFTRQFVANGQDLAKADGVLVNTFDALELEAVTALRQGSVAAGFP
PVFSVGPLVTVNLPAGEAAKESGNDYMYWLDAQPAQSVVYVSFGSRKAISRDQVRELAIGLEASGQPFLWVVKSTVVDR
DDSAELSELLGEGFLERVQGQGFVTKAWVEQEEVLKHESIGLFISHCGWNSVTEAAASGVPVLAWPRFGDQRVNAGVVA
RGGLGVWVERWSWEGEEDVVSGEEIAEKVKAVMADETVRKKAASVGEAAVKEFADGGTSYRSLTEFVRRCRDLAVLD*
> PGCGT1 (SEQ ID NO: 2) 
MPTSGSRTGGRPHVILLPSAGMGHLVPFSRLAVALSSGHGCDVSVVAVLPTVSAAESRHLQGLLDASPAVRRLDL
HLAPFDESEFPGADPFFLRFEAMRRSAPLLGPLLADTGASALVTDIALASVVIPVAKEIHLPCYVLFTASAAMLSFCAYFPT
YLDTNAGRVVGDVDIPGVYRIPKASIPQALHDPNHLFTRQFVANGQDLAKADGVLVNTFDALELEAVTALRQGSVAAGFP
PVFSVGPLVTVNLPAGEAAKESGNDYMYWLDAQPARSVVYVSFGSRKAISRNQVRELAVGLEASGQRFLWVVKSTVVD
RDDSAELSELLDEGFLERVQGRGLVTKAWVEQEEVLKHESIGLFISHCGWNSVTEAAASGVPVLAWPRFGDQRVNAGVV
ARGGLGVWVERWSWEGEEGVVSGEEIAEKVKAVMADETVRKKAASVGEAAVKEFADGGTSYRSLTEFVRRCRDLAVL
D*
> PpCGT1 (SEQ ID NO: 3) 
MPTSGSRTGGRPHVILLPSAGMGHLVPFSRLAVALSSGHGCDVSVVAVLPTVSAAESRHLQGLLDASPAVRRLDL
HLAPFDESEFPGADPFFLRFEAMRRSAPLLGPLLADTGESALVTDIALASVVIPVAKEIHLPCYVLFTASAAMLSFCAYFPTY
LDTNAGRVVGDVDIPGVYRIPKASIPQALHDRNHLFTRQFVANGQDLAKADGVLVNTFDALELEAVTALRQGSVAAGFPP
VFSVGPLVTVNLPAGEAAKESGNDYMYWLDAQPARSVVYVSFGSRKAISRNQVRELAVGLEASGQRFLWVVKSTVVDR
DDSAELSELLDEGFLERVQGRGLVTKAWVEQEEVLKHESIGLFISHCGWNSVTEAAASGVPVLAWPRFGDQRVNAGVVA
RGGLGVWVERWSWEGEEGVVSGEEIAEKVKAVMADETVRKKAASVGEAAVKEFADGGTSYRSLTEFVRRCRDLAVLD*
> PhCGT2 (SEQ ID NO: 4) 
MASRAMPSSSDRTGGAPRVVLLPSAGMGHLVPFGRLAVALSSGHACDVSVVTVLPTVSSAESKHLQGLFDAFPAV
RRLDLHLAPFDASEFSDADPFYVRYEAVRRSAPLLGPLLADADASALVADIALASVVIPVAKELLIPCYIFFTASATMFSLL
AYFPTYLHSNAGHSIGDVDVPGVYRIPMSSIPQALHDPNNLFTQQFVANGRSLAEADGLLVNTFDAFEPEAVTALRHGTVA
PGFPPVFALGPLSPVGFPAGEAARESGNYTTWLDAQPARSVVYVSFGSRKALLRDQLSELAAGLEASGCRFLWVVKGTVV
DRDDDRELRELLGEVFLQRVNGRALVTKAWVEQEEVLKHPAVGLFISHCGWNSVTEATVSGVPVLAWPRFADQRVSAG
VVARRGLGVWVERWSWEGEERVVTGDEIAEKVKSVLADETLRKNSTMVREAAAAAVATGGTSYRSLAKFVRRCST*
> OsCGT2 (SEQ ID NO: 5) 
MCSAATPNSGDVRATPGSSRPHVVLLPSAGMGHLVPFTRLAAALCSGHGCDVSLVAAVPTVSSAEARHLAAHFTA
FPAVRRLELDLASLDVSEFAGADPFYVRYEAIRRSASLLAPLLAGGASAAASALVADIALASVVIPVAKDLRLPCYVFFTAS
ATMFSFLAYLPTYLDANAGGGHAIGDVDVPGVCRVPTSSVPQALHDPDDIFTRQFIANARSLANADGLVVNAFDALEPEA
VAALRQGTVAAGLPPVFAVGPLSPAPIPAKDSGSYLPWLDAQPARSVVYVSFGSRKALPRDQLSELAAGLEASGHRFLWV
 
VKGAVVDRDDAGELTDLLGEAFLQRIHGRGLVTMAWVRQEEVLNHPSVGLFISHCGWNSVTEAAASGVPVVAWPRFAD
QRVNAGVVARAGIGVWVDTWSWEGEDDGVVSAEDIAGKVRSAMADEGVRKAAASVREAAARAVAAGGSSYRSLAEL
VRRCRDGLVITNGM*
> SbCGT1 (SEQ ID NO: 6) 
MAPPATKSLGELDGGARPHVMFIPSAGMGHLLPFFRVIAALAAHDVVDISVVTVLPTVSAAEADHFASLFAALPRV
SRVDFHLLPFDASSEFPGHDPFLLRWEALRRSAHLFRPLIAGAAGPRVSAVVTDVTLTSHVIPIAKELGVQCHVLFVSCATM
LSLAAYTPVHLDKKNKGEHGPGVGVGVGVGDVDIPGVRRIPQSYLPQPLLDLNKLFTKQFIDNGREIINADGFLVNTFDAL
EPVALAALRDGKVVAGFPPVYAIGPLRSKEEEATTGSPPVAWLDEQPARSVVYVAFGNRNAVSLEQIREIAAGLEASGCRF
LWVLKTTTVDRDDTAELTDDVLGEGFLERVQGRGLVTKAWVDQEAVLKHASVGLFLSHSGWNSVTEAAAAGVPLLAW
PRGGDHRVNATVVVSGGVGVWMEQWSWDGEDWLVTGEEIGKKVKEVMSDAAVRARATRTGEEAAKAVAEGGTSYRS
MQKFISSLKAHCSP*
> SbCGT2 (SEQ ID NO: 7) 
MAPSAMPASGDHTGATPHVVLLPSAGMGHLVPFARLAVALSEGHGCDVSFAAVLPTVSSAESRHLRALFAAAPA
VRRLDFRLAPFDESQFPGADPFFLRFEALRRSAPLLGPLLDAAQASALVTDIVLASVVLPVAKARGVPCYVLFTSSAAMLS
LCAYFPAHLDANAAAGGGRLGVGDVDIPGVYRIPKSSVPQALHDPKHLFTQQFVANGRGLVAADGILVNTFDAFEPDAIT
ALRQGSVVSDFPPVFAVGPLQPVRFQVAEKPAHYMRWLSAQPARSVVYVSFGSRKAISTDQLRELAAGLEASGQRFLWV
VKSTVVDRDDAADLADLLGDGFLERVQGRAFVTKGWVEQEEILQHGSVGLFISHCGWNSVTEAAAFGVPVLAWPRFGD
QRVNAAVVARGGLGAWEERWTWDGEKGLVTGEEVAEKINAVVGNDVVARKAARVGDAAAAAAGKGGTSYQSLADFV
GRCRDAGW*
> TaCGT1-A (SEQ ID NO: 8) 
MPTSGDGPTSQPHVVLLPSAGMGHLVPFSRLAVALSSAHGCDVSLVTVLPTVSSAESSHLEALFGAFPAVRRLEFH
LADFDASEFPNADPFFLRFEAMRRSAPLLLGPLLARASATALVTDIALSSVVIPVAKQLRLPCYVLFTASAAMLSLCVHFPA
YLDANGNGLVGDVDIPGVYQIPKASVPQALHDPKHLFTRQFVANGRELAKSDGVLVNSFDAFEPEAIAALREGAVSAAGF
FPPVFSVGPLAPVSFPAGNNNRADYIQWLEAQPARSVVYVSFGSRKAVARDQLRELAAGLEASGHRFLWVVKSTVVDRD
DDADLGELLGEGFLERVQGRGMVTKGWVEQEDVLKQESVGLFISHCGWNSVTEAAAGGLPVLAWPRFGDQRVNAGVV
ARSGLGVWVDSWSWEGEEGVVSGESIAEKVKAVMGDEIARNKAVSVRDAAAKAVADGGTSYRNLARFAQRCRDGRVR
E*
> TaCGT1-B (SEQ ID NO: 9) 
MPTSGDGSGALPHVVLLPSAGMGHLVPFSRLAVSFSSSDHGCGVSVVTVLPTVSSAESAHLDALFGACPAVRRLDF
HLARFDASEFPGADPFFLRFEAMRRSAPLLGPLLAGAGASALVTDIALASVVIPVARELRLPCYVLFTASAAMLSLCVHFPA
YLDANAGGPVGDVDIPGVYRVPKASIPQALHHPEHLFTRQFVANGRELAKSDGLLVNSFDEFEPEAISALRDGSVAAGFPP
VFSVGPLAPVSFSAGEPPENQADYMRWLEAQPTRSVVYVSFGSRKAISKDQLKELAVGLEASGHRFLWVVKSTVVDRED
EAELSELLGEGFLERVHGRGMVTKGWVEQEEVLKQESIGLFISHCGWNSVTEAAANGLPVLAWPRFGDQRVNAGVVARS
GLGVWEERWSWEGEEGVVSGENIAEKVKAVMADETVRNKAVSVQDAAAKAVADGGTSYRSLAQFVQRCRDLSVSK*
> TaCGT1-D (SEQ ID NO: 10) 
MPTSGDASGALPHPHVVLLPSAGMGHLVPFSRLAVSLSSPGHGCGVSVATVLPTVSSAESAHLDALFGACPAVRRL
DFHLAPFDASEFPGADPFFLRFEAMRRSAPLLGPLLAGAGASALVTDIALASVVIPVARDLRLPCYVLFTASAAMLSLCVHF
PAYLDANAGGPVGDVGIPGVYRVPKASIPQALHHPEHLFTQQFVANGRELAKADGLLVNSFDAFEPEAIAALRNGSVAAG
FPPVFSVGPLAPVSFSASEPPENQADYMRWLEAQPARSVVYVSFGSRKAISKDQLRELAVGLEASGHRFLWVVKSTVVDR
DDEAELSELLGEGFLERVQGRGMVTKGWVEQEEVLKQESIGLFISHCGWNSVTEAAANGLPVLAWPRFGDQRVNAGVV
ARSGLGVWEERWSWEGEEGTVSGHNIAEKVKTVMADKTVRNKAVSVQDAAAEAVADGGTSYRSLAQFVQRCRDLSVS
K*
> TaCGT2-A (SEQ ID NO: 11) 
MASSSRDDRAGAAAPRPRIVLLPSAGMGHLAPFSRLAAALSSGHACDVSLVTVLPTVSSAESGQLDALFAAFPAVR
RLDFHLPPLDAPELSGADPFYVHYEATRRSARLLAPLLAAADASALIADISLASVLIPVASELRLPCYVFFTASATMFSFYAY
YPTYLDATGAGDADVPGVYRIPKSSFPQALHDRNNLFTQQFVANGRSLSKADGLLINTFDDLEPEAVTALRQGSVVPGFPP
VFTVGPLSPVSFPARASSDYSAWLDAQPERSVVYVSFGSRKALARDQLSELADGLEASGCRFLWVVKGAVVDKDDGAEL
SELLGEGFLQRVSGRALVTKAWVEQGEVLKHPAIGMFVSHCGWNSLTEAFATGVPVLAWPRFADQRVNAGIVARCGAG
VWVERWSWEGDDGVVKGEEIAEKVKSVMADEMLRRSSTVVREAATTAVAGGGTSYRSLAELVARCCHGSALQ*
> TaCGT2-B (SEQ ID NO: 12) 
MASSPRDDRAGAAAPRPRIVLLPSASMGHLAPFSRLAAALSSGHACDVSLVTVLPTVSSAESGQLDALFAAFPAVR
RLDFQLPPLDAPELSGADPFYVHYEATRRSARLLAPLLAAADASVLVADISLASVLIPVASELRLPCYVFFTASATMFSFYA
YYPTYLDAAGAGDADVPGVYRIPKSSFPQALHDRNNLFTQQFVANGRSLSKADGLLINTFDALEPEAVTALRQGSVVPGC
PPVFTVGPLSPVSFPARASADYSACLDAQPERSVVYVSFGSRKALARDQLSELADGLEASGCRFLWVVKGAVVDKDDGA
ELSELLGEGFLQRVSGRALVTKAWVEQGEVLKHPAIGMFVSHCGWDSLTEAFATGVPVLAWPRFADQRVNAGIVARCGA
GVWVERWSWEGDEGVVKGEEIAEKVKSVMANEMLRRSSTMVREAAMTAVAGGGTSYRSLAELVARCCDGSASQ*
> TaCGT2-D (SEQ ID NO: 13) 
MASSSRDDRAGAAAPRPRIVLIPSAGMGHLAPFSRLAAALSSGHTCNVSLVTVLPTVSSAESGHLDALFAAFPAVR
RLDFQLPPLDAPELSGADPFYVHYEATRRSARLLAPLLAAADASALVADISLASVLIPVASELRLPCYVFFTASATMFSFYA
YYPTYLDAAGAGDADVPGVYRIPKSSFPQALHDRNNLFTQQFVANGQSLSKADGLLINTFDALEPEAVTALRQGSVVPGF
PPVFNVGPLSPVSFPARASSDYSAWLDAQPERSVVYVSFGSRKALARDQLSELADGLEASGCRFLWVVKGTVVDKDDGA
ELSELLGEGFLQRVSGRALVTKAWVEQGEVLKHPAIGMFVSHCGWNSLTEAFASGVPVLAWPRFADQRVNAGIVARRGA
GVWVERWSWEGDDGVVKGEEIAEKVKSVMADEMLRRSSTVVREAAMTAVAGGGTSYRSLAELVARCCDGSASNAPSE
 A*
>ZmCGT4 (SEQ ID NO: 14) 
MSSPAPSSASRSSHGSRGSPRIVLFPSAGMGHLVPFTRLAVALSAGHGCEISLLTALPTVSSAESRHIAALYAAFPAIR
QLDLRFAPFDASSEFPGADPFYLRYEALRRCAPSLLGPLLAGAGASALVVDMALASVAIPVARELHVPCFVFFTASATMLS
FKAYFPTYLDDVGAGHGVGDVDVPGVYRIPSSSVPQALHDPDNIFTRQFVANGRALATADGLLVNAFHAMEPEAVEALQ
GRFVLSVLPPVFAVGPLMPVNDLRETGEAAQKQGNYRAWLDEQPPRSVVYVSFGSRKALPKDQIKELAAGLEACGHRFL
WVVKGAVVDRDDAGELSELLGEAFLRRVHGRGLVTKSWVEQEEVLRHPAVALFVSHCGWNSVTEAVSSGVPVLAWPRF
ADQRVNARVVVRCGLGVWADQWSWEGEEALVRAEEITALVMEAMEDDAMAVKAANVREAASRAVVDGGTSYRSLAA
FVRRCTA*
> ZmCGT5 (SEQ ID NO: 15) 
MSSPAPSSPSRSSHGSRSSPRIVLFPSAGMGHLVPFTRLAVALSAGHGCDISLVTALPTVSSAESRHIAALYAAFPAIR
QLDLRFAPFDASSEFPGADPFYLRYEALRRCAPSLLGPLLAGAGASALVVDMALASVAIPVARELHVPCFVFFTASATMLS
FKAYFPTYLDDVGAGHGVGDVDVPGVYRIPSSSVPQALHDPDNIFTRQFVANGRALATADGLLVNAFHAMEPEAVEALQ
GRFVLSVLPPVFAVGPLMPVNDLRETGEAAQKQGNYRAWLDEQPPRSVVYVSFGSRKALPKDQIKELAAGLEACGHRFL
WVVKGAVVDRDDAGELSELLGEAFLRRVHGRGLVTKSWVEQEEVLRHPAVALFVSHCGWNSVTEAVSSGVPVLAWPRF
ADQRVNARVVVRCGLGVWADQWSWEGEEALVRAEEITALVMEAMEDDAMAVKAANVREAASRAVVDGGTSYRSLAA
FVRRCTA*
 >BdCGT1 (SEQ ID NO: 16) 
MPTSGDGPTSQPHVVLLPSAGMGHLVPFSRLAVALSSAHGCDVSLVTVLPTVSSAESSHLEALFGAFPAVRRLEFH
LADFDASEFPNADPFFLRFEAMRRSAPLLLGPLLARASATALVTDIALSSVVIPVAKQLRLPCYVLFTASAAMLSLCVHFPA
YLDANGNGLVGDVDIPGVYQIPKASVPQALHDPKHLFTRQFVANGRELAKSDGVLVNSFDAFEPEAIAALREGAVSAAGF
FPPVFSVGPLAPVSFPAGNNNRADYIQWLEAQPARSVVYVSFGSRKAVARDQLRELAAGLEASGHRFLWVVKSTVVDRD
DDADLGELLGEGFLERVQGRGMVTKGWVEQEDVLKQESVGLFISHCGWNSVTEAAAGGLPVLAWPRFGDQRVNAGVV
ARSGLGVWVDSWSWEGEEGVVSGESIAEKVKAVMGDEIARNKAVSVRDAAAKAVADGGTSYRNLARFAQRCRDGRVR
E*
> SCGT1 (SEQ ID NO: 17) 
MPTSGDHSGARPHVVLLPSAGMGHLVPFGRLAVALSAGHGCDVSVAAVLPTVSSAEARHLEALFAAADPAVRRL
DFRLAPFDASEFPGADPFFLRFEAMRRSAPLLGPLLATAGASAVVTDIVLASVVLPVARECGVPCYVLFTASAAMLAFCAH
FPGYLDANSAAGRAGVVGDVDIPGVYRIPKSSVPQALHDPKHLFTQQFVANGRGLVDADGILVNTFDALEPEAVTALRKG
KVVSSFPLVFAVGPLLPVMFPAKDPAGYMQWLDAQPARSVVYVSFGSRTAISPDQLLELAAGLEASGHRFLWVVKSTVV
DRDDAGELGDLLGHGFLERVRGRALVTKGWVEQEEILQHGSVGLFVSHCGWNSVTEAAAFGVPVLAWPRFGDQRINAA
VVTRGGLGAWEERWSWDGEEGLVSGEEVGEKIKAVMADETVAKKAAAVGDAAAAAATSKGGTSYRSLAEFVGRCRDA
GSRQDR*
> SiCGT2 (SEQ ID NO: 18) 
MSSPPPPNSTSDDCQGTAATPHIVLFPSAGMGHLVPFTRLAVALSAGHCCDVSLVTALPTVSSAESCHIAALFAAFP
AVRRLDLRLAPLDASSEFPGADPFYVRYEALRRSTPLLLGPLLAGASASALVADIALASVAIPVARELRVPCYVFFTASATM
LSFKAYFPTYLDANGGAGHSVGDVDVPGVCRVPRSSIPQALHDPDDIFTRQFVANGRALVAADGLLVNAFEAMEPEAVA
ALLGGSVVASLPPVFAVGPLMPLNLREAAEEQGNYRAWLDAQPPRSVVYVSFGSRKALARDQIRELAAWLEACGHRFLW
VVKGAVVDRDDACELSDLLGEGFQRRVEGRGLVTKSWVEQDEVLRHPAVGLFVSHCGWNSVTEAAANGVPVLAWPRF
ADQRVNARVVARAGFGVWVERWSWEGEEAVVGAEEIAEQVVAAMGDQAVAEKAASVRDEAARAVADGGTSQQSLAE
FVRRCRGGLAGPGTDLGRTWTARG*
> OsCGT (SEQ ID NO: 19) 
MPSSGDAAGRRPHVVLIPSAGMGHLVPFGRLAVALSSGHGCDVSLVTVLPTVSTAESKHLDALFDAFPAVRRLDF
ELAPFDASEFPSADPFFLRFEAMRRSAPLLGPLLTGAGASALATDIALTSVVIPVAKEQGLPCHILFTASAAMLSLCAYFPTY
LDANAGDGGGVGDVDIPGVYRIPKASIPQALHDPNHLFTRQFVANGRSLTSAAGILVNTFDALEPEAVAALQQGKVASGF
PPVFAVGPLLPASNQAKDPQANYMEWLDAQPARSVVYVSFGSRKAISGEQLRELAAGLETSGHRFLWVVKSTVVDRDDA
AELGELLGEGFLKRVEKRGLVTKAWVDQEEVLKHESVALFVSHCGWNSVTEAAASGVPVLALPRFGDQRVNSGVVARA
GLGVWADTWSWEGEAGVIGAEEISEKVKAAMADEALRRKAASLAKAAAKAVAGGGSSHRCLVEFARLCQGGTCRTN*
> ZmCGT (SEQ ID NO: 20)

MAANGGDHTSARPHVVLLPSAGMGHLVPFARLAVALSEGHGCNVSVAAVQPTVSSAESRLLDALFVAAAPAVRR
LDFRLAPFDESEFPGADPFFLRFEATRRSAPLLGPLLDAAEASALVTDIVLASVALPVARERGVPCYVLFTSSAAMLSLCAY
FPAYLDAHAAAGSVGVGVGNVDIPGVFRIPKSSVPQALHDPDHLFTQQFVANGRCLVACDGILVNTFDAFEPDAVTALRQ
GSITVSGGFPPVFTVGPMLPVRFQAEETADYMRWLSAQPPRSVVYVSFGSRKAIPRDQLRELAAGLEASGKRFLWVVKSTI
VDRDDTADLGGLLGDGFLERVQGRAFVTMGWVEQEEILQHGSVGLFISHCGWNSLTEAAAFGVPVLAWPRFGDQRVNA
ALVARSGLGAWEEGWTWDGEEGLTTRKEVAKKIKGMMGYDAVAEKAAKVGDAAAAAIAKCGTSYQSLEEFVQRCRD
AERK*

Using genomic DNA as a template, the following C-glycosyltransferase genes were amplified by PCR: PhCGT1 (SEQ ID NO: 1), PgCGT1 (SEQ ID NO: 2), PpCGT1 (SEQ ID NO: 3), PhCGT2(SEQ ID NO: 4), OsCGT2(SEQ ID NO: 5), SbCGT1 (SEQ ID NO: 6), SbCGT2(SEQ ID NO: 7), TaCGT1-A(SEQ ID NO: 8), TaCGT1-B(SEQ ID NO: 9), TaCGT1-D (SEQ ID NO: 10), TaCGT2-A(SEQ ID NO: 11), TaCGT2-B(SEQ ID NO: 12), TaCGT2-D (SEQ ID NO: 13), ZmCGT4(SEQ ID NO: 14), ZmCGT5(SEQ ID NO: 15), BdCGT1 (SEQ ID NO: 16), SiCGT1(SEQ ID NO: 17), SiCGT2(SEQ ID NO: 18), OsCGT(SEQ ID NO: 19), ZmCGT(SEQ ID NO: 20). PCR primers are shown in Table 1. Among them, PhCGT1, PgCGT1, and PpCGT1 were amplified using the same primer pair (Ph708UGT1-F/Ph708UGT1-R); TaCGT1-B, TaCGT2-B, SiCGT1, and ZmCGT were artificially synthesized and codon-optimized based on *E. coli.*

**Table 1. Primers used for cloning glycosyltransferases**

| Gene Name | Primer Name | Sequence 5'-3' (SEQ ID NO:) |
|---|---|---|
| *PhCGT1, PgCGT1, PpCGT1* | Ph708UGT1-F | tgccgcgcggcagccatatgatgggccacctggtgc (21) |
| | Ph708UGT1-R | tggtgctcgagtgcggccgcctagtccaacactgcaagatccc (22) |

| *PhCGT2* | Synthetic | |
|---|---|---|
| *OsCGT2* | Os708A4-F | tgccgcgcgoca,gccatatgatgtgttcggcggcaacac (23) |
| | Os708A4-R | tgetgctcgagtgcggccgctcacattccgttcgtgatgacaagc (24) |
| *SbCGT1* | Sb2-F | tgccgcgcggcagccatatgatggccccatcggcgat (25) |
| | Sb2-R | tgetgctcgagtgcggccgcctaccaccccgcgtcgc (26) |
| *SbCGT2* | Sb708UGT-4 F | tgccgcgcggcagccatatgatgtcctcgccggcattatca (27) |
| | Sb708UGT-4 R | tgetgctcgagtgcggccgctcacgccgtgcaacgac (28) |
| *TaCGT1-A* | TalA F | tgccgcgcggcagccatatgatgccgacctccggcg (29) |
| | TalA R | tgetgctcgagtgcggccgcttattccgtgttcacgcttcttctaaatgtagc (30) |

| *TaCGT1-B* | Synthetic | |
|---|---|---|
| *TaCGT1-D* | TalA F | tgccgcgcggcagccatatgatgccgacctccggcg(31) |
| | TalD R | tggtgctcgagtgcggccgcttacttgctgacgcttagatcccgg(32) |
| *TaCGT2-A* | Ta2A-F | tgccgcgcggca,gccatatgatggcctccagctcgagagac(33) |
| | Ta2A-R | tggtgctcgagtgcggccgcttactgtaaggcactaccatgacagcag(34) |

| *TaCGT2-B* | Synthetic | |
|---|---|---|
| *TaCGT2-D* | Ta2D-F | tgccgcgcggcagccatatgatggcctccagctccagag(35) |
| | Ta2D-R | tgat,gctcgagtgcg.gccgctcacgcctctgacg.gcg(36) |
| *ZmCGT4* | Zm708UGTS2-F | tgccgcgcggcagccatatgatgtcctcgccggcac(37) |
| | Zm708UGTS2-R | tggtgctcgagtgcggccgctcatgcggtgcaacgacg(38) |
| *ZmCGT5* | Zm708UGTS3-F | tgccgcgcggcagccatatgatgtcctcgccggcac(39) |
| | Zm708UGTS3-R | tggtgctcgagtgcggccgcttatataacttcacgcggtgcaatgc(40) |
| *BdCGT1* | Bd708A7-F | tgccgcgcggcagccatatgatgccgacctccggcg(41) |
| | Bd708A7-R | tggtgctcgagtgcggccgcttactcacgcactcttccatcccg(42) |

| *SiCGT1* | Synthetic | |
|---|---|---|
| *SiCGT2* | Si2-F | tgccgcgcggcagccatatgatgtcctcaccgccaccg(43) |
| | Si2-R | tggtgctcgagtgcggccgctcatcctcgcgctgtccacg(44) |
| *OsCGT* | Os708A3-F | tgccgcgcggcagccatatgatgccgagctctggcga(45) |
| | Os708A3-R | tggtgctcgagtgcggccgctcaattagtgcgacatgttccccc(46) |
| *ZmCGT* | Synthetic | |

PCR amplification system 25µL (PrimeSTAR Max Premix, 12.5µL; Primer 1/Primer 2 with final concentration of 0.2µM; gDNA<50ng; the remaining volume is supplemented with sterilized distilled water). PCR reaction conditions: pre-denaturation at 98 °C for 2 min, then denaturation at 98 °C for 10 s, annealing at 55 °C for 15 s, extension at 72 °C for 10 s, 30 cycles, extension at 72°C for 10 min, agarose electrophoresis detection, amplification to obtain a fragment of about 1.5kb. After purification, the fragment was ligated into the *NdeI*/*NotI* digested pET28a vector by seamless cloning, and the ligation product was transformed into *E. coli* DH10B competent cells, and the sequence was confirmed by sequencing.

### 2. Protein expression of C-glycosyltransferase

The recombinant plasmid was transformed into competent cells of *Escherichia coli* BL21 (DE3), and cultured overnight at 37°C in solid LB medium (kanamycin 50 µg/mL). Single colony was transferred to a 2 mL LB liquid medium (containing 50 µg/ml kanamycin) and incubated overnight. The bacterial fluid was transferred to a new 100 ml LB liquid medium with antibiotics and incubated at 37 °C and 250 r/min until OD₆₀₀ reached 0.5-0.6. The culture was cooled down to 16 °C in a water bath. Then inducer IPTG was added at a final concentration of 0.1 mM. The mixture was cultured at 16 °C for low temperature induction and 180 r/min for 20 h. The BL21(DE3) recombinant strain carrying the pET28a empty plasmid was used as a blank control, and the culture operation was the same as above. The bacteria were harvested by centrifugation (6000 r/min, 5 min), re-suspended in 1 mL of lysis buffer Buffer A (20 mM Tris-HCl, pH 8.0; 100 mM NaCl), and DNase was added to a final concentration of 5 µg/mL, Mg²⁺ to a final concentration of 2 mM; protease inhibitor PMSF to a final concentration of 1 mM; the bacteria were incubated on ice for 30 min and then sonicated; refrigerated centrifugation (10,000 rpm, 30 min, 4 °C), filtered through a 0.2 µm filter membrane to obtain the supernatant crude enzyme solution, and added 20% glycerol for cryopreservation (- 20°C).

### 3. In vitro functional identification of C-glycosyltransferases

The *in vitro* enzyme catalytic assay system is 100 µL. The reaction system includes: 0.1 mM glycosyl receptor (phloretin or 2-hydroxynaringenin), 0.3 mM UDP glucose, 25 µL supernatant crude enzyme solution, and Buffer A is used to make up the volume to 100 µL, and react at 37 °C for 2 h. After the reaction was completed, an equal volume of methanol was added to terminate the reaction, and HPLC was used for product analysis. HPLC detection conditions: chromatographic column: reverse phase C18 column [SilGreen ODS column (ø 4.6×250 mm)], detection wavelength 280nm; mobile phase: acetonitrile (0.1% formic acid)-water (0.1% formic acid) mixture gradient elution, with increased ratio from 5:95 to 100:0 within 0 to 20min, and the flow rate was 1mL/min.

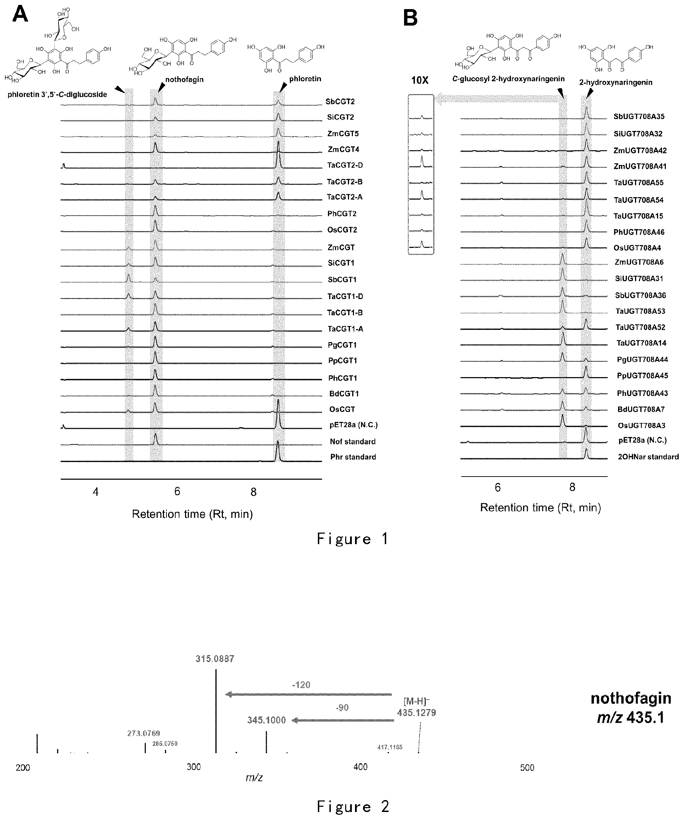

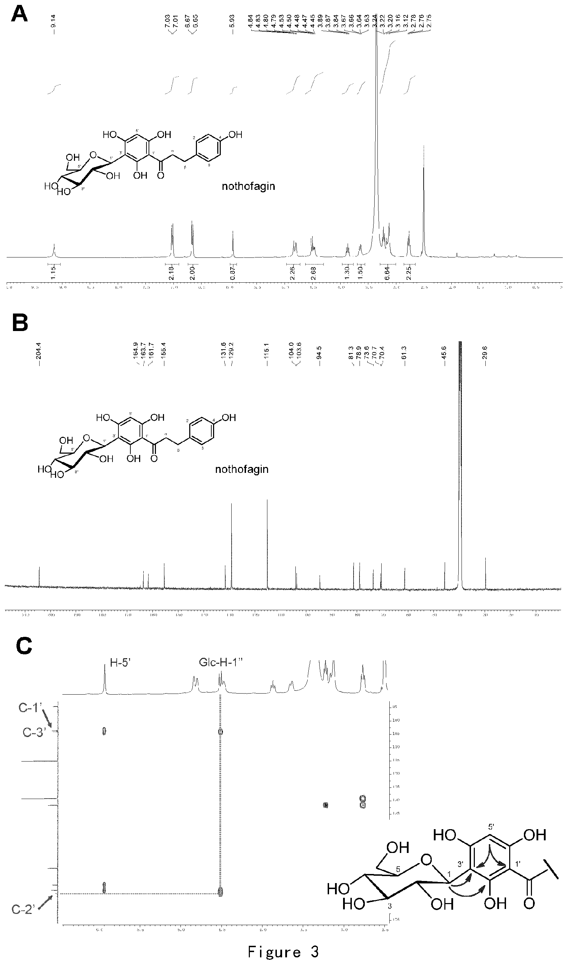

According to HPLC detection result 1 (Figure 1A), PhCGT1, PgCGT1, PpCGT1, PhCGT2, OsCGT2, SbCGT1, SbCGT2, TaCGT1-A,TaCGT1-B, TaCGT1-D, TaCGT2-A, TaCGT2-B, TaCGT2-D, ZmCGT4, ZmCGT5, BdCGT1, SiCGT1, SiCGT2, OsCGT, ZmCGT can convert the substrate phloretin to a product having a shorter retention time. The above product was confirmed to be C₂₁H₂₄O₁₀ by high-resolution mass spectrometry in negative ion mode, and the secondary mass spectrometry showed [M-H-90]⁻, [M-H-120]⁻ fragment ion peaks of hexose carbonoside characteristics (Figure 2). The carboglycoside glycosylated product was identified as Nothofagin by one-dimensional nuclear magnetic resonance (¹H NMR, ¹³C NMR) and two-dimensional nuclear magnetic resonance HMBC spectroscopy (Fig. 3).

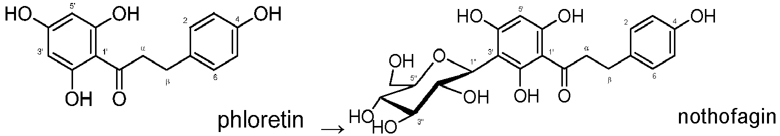

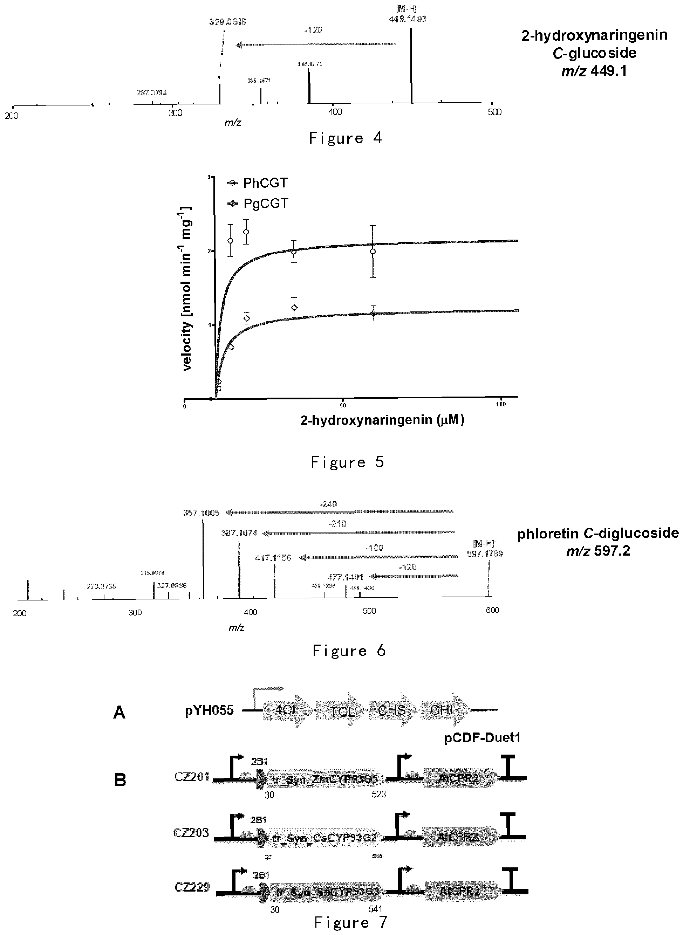

Meanwhile, the above 20 C-glycosyltransferases can convert the substrate 2-hydroxynaringenin into its glycosylation product (Fig. IB). The product was confirmed to be C₂₁H₂₂O₁₁ by high-resolution mass spectrometry detection in negative ion mode, and the secondary mass spectrometry showed [M-H-120]⁻ fragment ion peak of carbon hexoside characteristics (Figure 4), suggesting that the product was 2-hydroxynaringenin -C-glucoside.

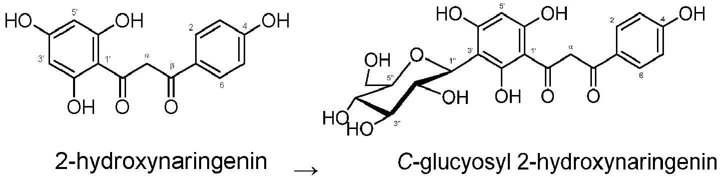

The inventors measured the kinetic parameters of the newly discovered C-glycosyltransferases, and found that the Km values of PhCGT1 and PgCGT1 for 2-hydroxynaringenin were the lowest among all C-glycosyltransferases, reaching 1.75 ± 0.66 and 2.75 ± 0.75 µM, respectively(FIG. 5). The *Km* value is the substrate concentration when the enzymatic reaction presents half of its maximum reaction rate, and the lower the *Km* value, the higher the enzymatic activity of the C-glycosyltransferase.

In addition, the inventors also found that SbCGT1, TaCGT1-A,TaCGT1-D, SiCGT1, OsCGT and ZmCGT can not only catalyze the glycosylation of phloretin at the 3' position, but also can catalyze the glycosylation of the 3'-glycosylated phloretin at the 5' position, generating a dihydrochalcone di-carboglycoside (phloretin-3',5'-*C*-diglucoside, Figure 1A). The above product was confirmed to be C₂₇H3₄O₁₅ by high-resolution mass spectrometry in negative ion mode, and the secondary mass spectrometry showed [M-H-180]⁻, [M-H-210]⁻, and [M-H-240]⁻ fragment ion peaks of dihexose carbonoside characteristics (Figure 6).

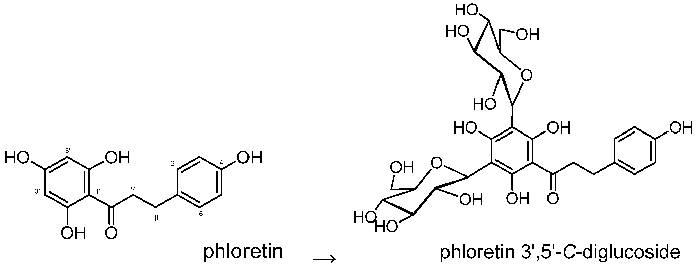

### Example 2. Synthesis of vitexin, isovitexin, orientin and isoorientin with glycosyltransferase of the present disclosure

In this example, PhCGT1 was used to synthesize vitexin, isovitexin, orientin and isoorientin in Escherichia coli.

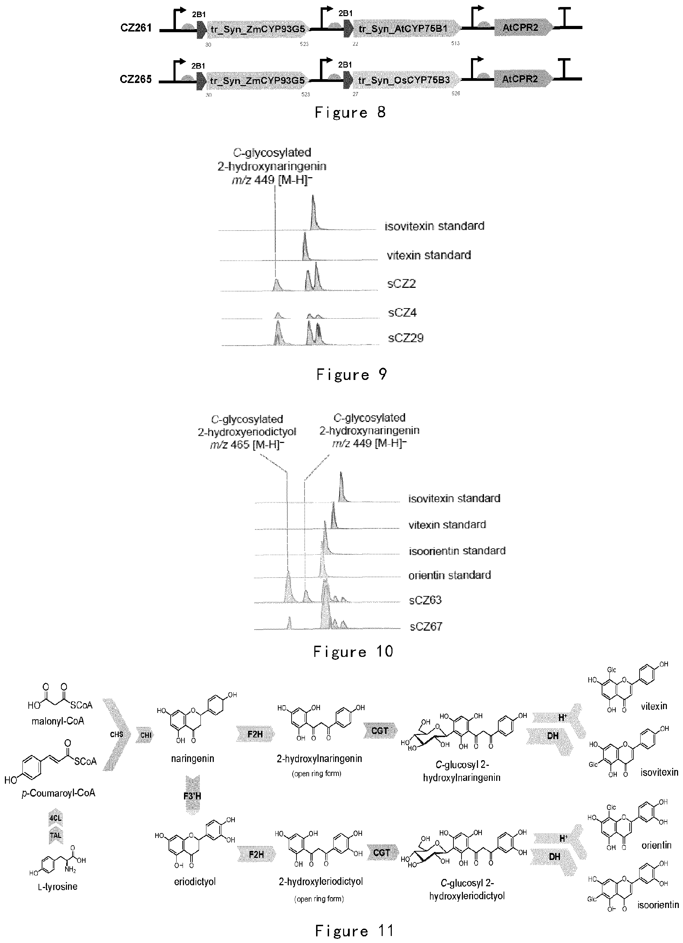

The predicted biosynthetic pathway of flavonoid-C-monoglucoside is shown in Figure 11. Arrows of TAL (or PAL), 4CL, CHS, CHI represent the major enzymes responsible for naringenin biosynthesis. Arrows of F2H, F3'H are flavanone 2-hydroxylase (F2H) and flavanone 3'-hydroxylase (F3'H), which constitute the backbone of hydroxylated flavanones. C-glycosyltransferase (CGT) is indicated by arrowed CGT. After the formation of C-glycosylation intermediates, the dehydration reaction occurs spontaneously in acidic solvents (arrow of H⁺) or catalyzed by dehydratase (arrow of DH).

### 1. Plasmid construction

### (1) Construction of the expression cassette of the synthetic precursor gene of the flavanone naringenin

The synthetic and codon-optimized precursor synthetic gene sequences were constructed into pET28a vector to obtain pET28-RtPAL, pET28-Pc4CL, pET28-PxhCHS and pET28a-MsCHI. PxhCHS: GenBank accession number KF765781; MsCHI: GenBank accession number KF765782; Pc4CL: GenBank accession number KF765780; RtPAL: GenBank accession number AAA33883.

*Pc4CL* fragment is amplified using pET28-Pc4CL as templates and 4CL-F-NcoI/4CL-R-BamHI as primers. pYH40 was constructed by ligation of the amplified products with *NcoI*/*BamHI* digested pCDFDuet-1.

*PxhCHS* fragment is amplified using pET28-PxhCHS as templates and CHS-F-NdeI/CHS-R-XhoI as primers. pYH50 was constructed by ligation of the amplified products with NdeI/XhoI digested pYH40.

*McCHI* fragment is amplified using pET28-MsCHI as templates and T7CHI-F-XhoI /CHI-R-AvrII as primers. pYH51 was constructed by ligation of the amplified products with *Xho*I/*Avr*II digested pYH50.

*RtPAL* fragment is amplified using pET28-RtPAL as templates and T7PAL-F-BamHI/PAL-R- HindIII as primers. pYH55 (pYH055) for naringenin *de novo* synthesis was constructed by ligation of the amplified products with *BamH*I/*Hind*III digested pYH51 (Fig. 7A). PCR primers are shown in Table 2.

**Table 2. Primers used for the construction of the expression cassette of naringenin synthesis precursor gene**

| | Primer | Sequence (SEQ ID NO:) |
|---|---|---|
| | 4CL-F-NcoI | tataccatgegtgactgcgttgccccg(47) |
| | 4CL-R-BamHI | cgggatccttacttcggcaggtcgccgctc(48) |
| | T7PAL-F-BamHI | cgegatcccttatgcgactcctgcattag(49) |
| | PAL-R-HindIII | gcccaagcttttatgccagcatcttc(50) |
| | CHS-F-NdeI | agatatacatatggttacggtggaagaatac(51) |
| | CHS-R-XhoI | ccgctcgagttaggtagccacactatgcag(52) |
| | T7CHI-F-XhoI | ccgctcgagctagaaataattttgtttaac(53) |
| | RbsCHI-F-XhoI | ccgctcgagcgatcccgcgaaattaatacg(54) |
| | CHI-R-AvrII | gagcctaggttagttaccgattttaaag(55) |

### (2) Construction of flavanone-2-hydroxylase/CPR expression cassette

Three P450 genes were amplified by PCR with the synthesized cloning vectors of flavanone-2-hydroxylase encoding gene *OsCYP93G2* (codon-optimized), *ZmCYP93G5* (codon-optimized) or *SbCYP93G3* (codon-optimized) as a template. The above three genes were modified at the N-terminal corresponding to their proteins, the N-terminal transmembrane region was truncated and the tag 2B1 (sequence: MAKKTSSKGKLPPGPS) was added.

OsCYP93G2: GenBank accession number XP_015642954.1, with N-terminal residues 1 to 26 truncated and 2B1 added, and its encoding gene was optimized for *E. coli*-preferred codons by conventional methods;

*ZmCYP93G5:* GenBank accession number XP_008660140.1, with N-terminal residues 1 to 29 truncated and 2B1 added, and its encoding gene was optimized for *E. coli*-preferred codons by conventional methods;

*SbCYP93G3:* GenBank accession number XP_002461286.1, with N-terminal residues 1 to 37 truncated and 2B1 added, and its encoding gene was optimized for *E. coli*-preferred codons by conventional methods;

The *AtCPR2* gene (GenBank Accession No. ABB88839.2, used in combination with P450 to provide reducibility) was amplified by PCR using Arabidopsis cDNA as a template.

The modified P450 gene was amplified using primer pairs CZ201-F/R, CZ203-F/R, and CZ229-F/R, respectively, and inserted between the *NcoI* and *BamHI* of multiple cloning site 1 of pDuet-1 vector by seamless cloning method. Meanwhile, AtCPR2 was amplified using the primer pair CPR-F/NotI-trCPR-R and inserted between the *NdeI* and *XhoI* sites of multiple cloning site 2 of the pDuet-1 vector to form plasmids pCZ201, pCZ203 and pCZ229, respectively (Figure 7B). PCR primers are shown in Table 3.

**Table 3. Primers used to construct pCZ201, pCZ203 and pCZ229**

| Gene | Primer | Sequence (5'→3') (SEQ ID NO:) |
|---|---|---|
| *ZmCYP93G5* | CZ201-F | agctcccaccaggacctagcatgagcacctggtccaaccg(56) |
| | CZ201-R | gagctcgaattcggatccactagtttaggtcgccgcacgcgcc(57) |
| *OsCYP93G2* | CZ203-F | ctcccaccaggacctagcatgcgtagcgcgggtagccg(58) |
| | CZ203-R | tcgaattcggatccactagtttagctataaaagctcggcagcg(59) |
| *SbCYP93G3* | CZ229-F | agctcccaccaggacctagcatgatcattcgttggcgttggaaca(60) |
| | CZ229-R | gagctcgaattcggatccactagtttaggtcgctttaccggtcgc(61) |
| *AtCPR2* | CPR-F | tatacatatgtcctcttcttcttcttcgtca(62) |
| | NotI-trCP R-R | aattgcggccgcttaccatacatctctaagat(63) |

### (3) Construction of flavanone-2-hydroxylase/flavanone-3'hydroxylase/CPR expression cassette

The P450 genes were amplified by PCR with the synthesized cloning vectors of flavanone-3'-hydroxylase *AtCYP75B1* (codon-optimized), or *OsCYP75B3* (codon-optimized) as a template. The genes were modified at the N-terminal, wherein the N-terminal transmembrane region was truncated and the tag 2B1 (sequence: MAKKTSSKGKLPPGPS) was added.

*AtCYP75B1:* GenBank accession number NP_196416.1, with N-terminal residues 1 to 21 truncated and 2B1 added, and its encoding gene was optimized for *E. coli*-preferred codons by conventional methods;

*OsCYP75B3:* GenBank accession number XP_015613041.1, with N-terminal residues 1 to 26 truncated and 2B1 added, and its encoding gene was optimized for *E. coli*-preferred codons by conventional methods;

The *AtCPR2* gene (GenBank Accession No. ABB88839.2, used in combination with P450 to provide reducibility) was amplified by PCR using Arabidopsis cDNA as a template.

The modified P450 gene was amplified with primer pairs CZ261-F/R and CZ265-F/R, respectively, and was connected with the better ZmCYP93G5 encoding gene of flavanone-2-hydroxylase by fusion PCR. The ligated binary P450 combination was inserted between *NcoI* and *BamHI* of multiple cloning site 1 of pDuet-1 vector by seamless cloning, and *AtCPR2* was amplified using the primer pair CPR-F/NotI-trCPR-R (Table 3) and inserted between the *NdeI* and *XhoI* sites of multiple cloning site 2 of the pDuet-1 vector to form plasmids pCZ261 and pCZ265, respectively (Figure 8). PCR primers are shown in Table 4.

**Table 4. Primers used to construct pCZ261 and pCZ265**

| Gene | Primer | Sequence (5'→3') |
|---|---|---|
| *AtCYP75B1* | CZ261-F | cgacctaaactagtggatcctaatacgactcactataggggaattgtgag(64) |
| | CZ261-R | cttaagcattatgcggccgcttaaccgctgcccagaccat(65) |
| *OsCYP75B3* | CZ265-F | cgacctaaactagtggatcctaatacgactcactataggggaattgtgag(66) |
| | CZ265-R | cttaagcattatgcggccgcttaaacgccatacgcgctcgg(67) |

### (4) Construction of PhCGT1 expression plasmid

*PhCGT1* was amplified by PCR (primers are shown in Table 1) using the genomic DNA of moso bamboo as a template. The amplified fragment was purified and ligated into the same *NdeI*/*NotI* digested pET28a vector by seamless cloning to form plasmid pCZ86.

### 2. Construction and fermentation of engineered bacteria for vitexin/isovitexin synthesis

The plasmid pYH55 carrying the naringenin synthesis gene and the plasmid pCZ86 carrying the novel glycosyltransferase PhCGT1 were co-transformed with the plasmids pCZ201, pCZ203 and pCZ229 carrying the flavanone-2-hydroxylase/CPR expression cassette, respectively, to *Escherichia coli* BL21 (DE3) (co-transformation of three plasmids), and the *de novo* fermentation and synthetic engineering strains sCZ2, sCZ4 and sCZ29 were obtained.

The single clone of above-mentioned engineering strain was selected and inoculated in 10mL MOPS medium (Spe=50 µ g/mL, Amp=100 µ g/mL, Kan=50 µ g/mL), cultivated to OD600=0.5 at 37 °C, cooled down to 22 °C, added with IPTG (final concentration of 100 µ M), and the fermentation was carried out at 22° C and 200 rpm for 5 days. After the cultivation, the samples were collected, and the bacterial liquid was centrifuged to obtain the supernatant and bacteria. The bacteria were suspended in water, crushed and then extracted with ethyl acetate and n-butanol; the supernatant was extracted with ethyl acetate and n-butanol, and the extracts were combined. The extract was evaporated and re-dissolved in methanol for LC-MS analysis.

The fermentation results show (Fig. 9), after 5 days of fermentation, vitexin and isovitexin can be detected in the fermentation broth, with the ratio of about 4:6. Meanwhile, the intermediate 2-hydroxynaringenin-C-glucoside (i.e., 2-hydroxynaringenin-6(8)-C-glucoside) can be detected in the fermentation broth.

Therefore, the C-glycosyltransferase (such as PhCGT1) of the present disclosure can specifically and efficiently catalyze the carboglycoside glucosylation of 2-hydroxyflavanone (opened-ring form) compounds (such as 2-hydroxynaringenin), thereby producing a type of carboglycosides-2-hydroxyflavanones type compounds (such as 2-hydroxynaringenin-C-glucoside). These compounds form C-glycosylated flavone compounds (such as vitex and isovitexin) through further dehydration reaction.

### 3. Construction and fermentation of engineered bacteria for orientin/isoorientin synthesis

The plasmid pYH55 carrying the naringenin synthesis gene and the plasmid pCZ86 carrying the novel glycosyltransferase PhCGT1 were co-transformed with the plasmids pCZ261 and pCZ265 carrying the flavanone-2-hydroxylase/flavanone-3'-hydroxylase/CPR expression cassette, respectively, to *Escherichia coli* BL21 (DE3) (co-transformation of three plasmids), and the *de novo* fermentation and synthetic engineering strains sCZ63 and sCZ67 were obtained.

The single clone of above-mentioned engineering strain was selected and inoculated in 10mL MOPS medium (Spe=50 µ g/mL, Amp=100 µ g/mL, Kan=50 µ g/mL), cultivated to OD600=0.5 at 37°C, cooled down to 22°C, added with IPTG (final concentration of 100 µ M), and the fermentation was carried out at 22° C and 200 rpm for 5 days. After the cultivation, the samples were collected, and the bacterial liquid was centrifuged to obtain the supernatant and bacteria. The bacteria were suspended in water, crushed and then extracted with ethyl acetate and n-butanol; the supernatant was extracted with ethyl acetate and n-butanol, and the extracts were combined. The extract was evaporated and re-dissolved in methanol for LC-MS analysis.

The fermentation results show (Fig. 10), after 5 days of fermentation, the mixture of orientin and isoorientin can be detected in the fermentation broth, with the ratio of about 4:6. Meanwhile, a certain proportion (about 20-25%) of vitexin and isovitexin without 3' hydroxylation can also be detected in the fermentation broth. Accumulation of intermediates 2-hydroxynaringenin-C-glucoside and 2-hydroxyeriodictyol-C-glucoside was detected in strain sCZ63.

Therefore, the C-glycosyltransferase (such as PhCGT1) of the present disclosure can specifically and efficiently catalyze the carboglycoside glucosylation of dihydrochalcone, 2-hydroxyflavanone (opened-ring form) compounds (such as 2-hydroxyeriodictyol), thereby producing a type of carboglycosides dihydrochalcone type compounds (such as 2-hydroxyeiodictyol-C-glucoside). These compounds form C-glycosylated flavone compounds (such as orientin and isoorientin) through further dehydration reaction.

### Example 3. Synthesis of vitzenin-2 with glycosyltransferase of the present disclosure

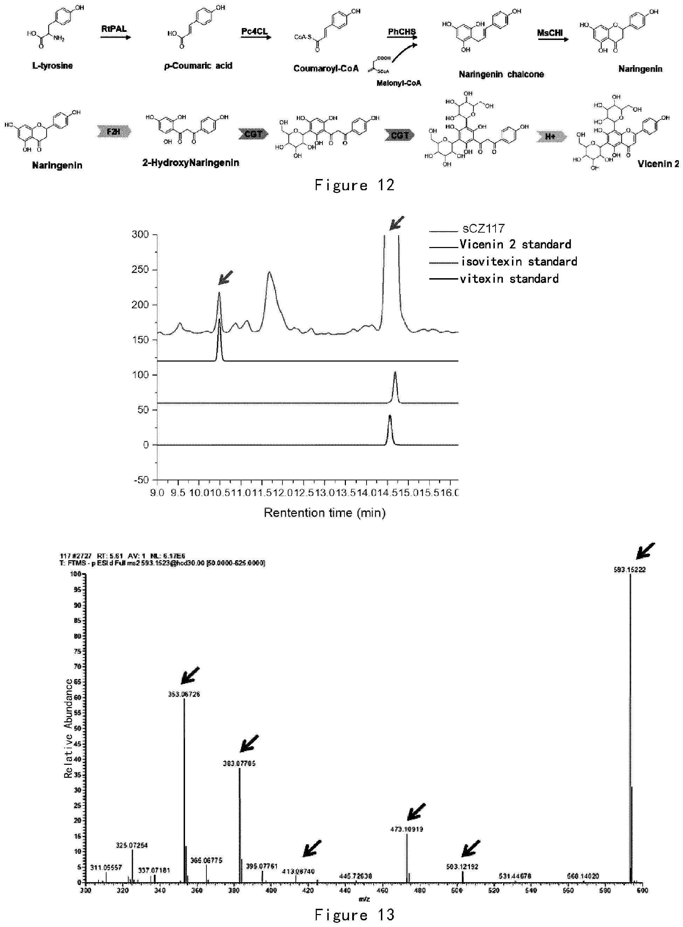

In this example, TaCGT1-D was used to synthesize vicenin-2 in E. coli. Figure 12 shows the predicted biosynthetic pathway of vitzenin-2 (apigenin-6,8-diglucoside). Arrows of TAL (or PAL), 4CL, CHS, CHI represent the major enzymes responsible for naringenin biosynthesis. Arrow of F2H is flavanone 2-hydroxylase (F2H), which constitutes the backbone of 2-hydroxyflavanone. C-glycosyltransferase (CGT) is indicated by arrow of CGT. After the formation of C-glycosylation intermediates, the dehydration reaction occurs spontaneously in acidic solvents (arrow of H⁺).

### 1. Plasmid construction

(1) Construction of the expression cassette of the synthetic precursor gene of the flavanone naringenin is according to Example 2.
(2) Construction of flavanone-2-hydroxylase/CPR expression cassette is according to Example 2.
(3) Construction of TaCGT1-D expression plasmid: TaCGT1-D was amplified by PCR using wheat genomic DNA as a template (see Table 1 for primers). The amplified fragment was purified and ligated into the same *NdeI*/*NotI* digested pET28a vector by seamless cloning to form plasmid pCZ173.

### 2. Construction and Fermentation of Engineered Bacteria for Vitzenin-2 Synthesis

The plasmid pYH55 carrying the naringenin synthesis gene and the plasmid pCZ173 carrying the novel glycosyltransferase TaCGT1-D were co-transformed with the plasmids pCZ201 carrying the flavanone-2-hydroxylase/CPR expression cassette, respectively, to *Escherichia coli* BL21 (DE3) (co-transformation of three plasmids), and the *de novo* fermentation and synthetic engineering strain sCZ117 was obtained.

The single clone of above-mentioned engineering strain was selected and inoculated in 10mL MOPS medium (Spe=50 µ g/mL, Amp=100 µ g/mL, Kan=50 µ g/mL), cultivated to OD600=0.5 at 37 °C, cooled down to 22°C, added with IPTG (final concentration of 100 µ M), and the fermentation was carried out at 22° C and 250 rpm for 5 days. After the cultivation, the samples were collected, and the bacterial liquid was centrifuged to obtain the supernatant and bacteria. The bacteria were suspended in water, crushed and then extracted with ethyl acetate and n-butanol; the supernatant was extracted with ethyl acetate and n-butanol, and the extracts were combined. The extract was evaporated and re-dissolved in methanol for LC-MS analysis.

The fermentation results showed that after 5 days of fermentation, Vitzenin 2 could be detected in the fermentation broth (Fig. 13), and meanwhile intermediate vitexin and isovitexin also could be detected in the fermentation broth.

Therefore, the C-glycosyltransferase (such as TaCGT1-D) of the present disclosure can specifically and efficiently catalyze the di-carboglycoside glucosylation of 2-hydroxyflavanone (opened-ring form) compounds (such as 2-hydroxynaringenin), thereby producing a type of di-carboglycosides-2-hydroxyflavanones type compounds (such as 2-hydroxynaringenin-C-diglucoside). These compounds form di-carboglycoside flavonoid compounds (such as vitzenin 2) through further dehydration reaction.

Each reference provided herein is incorporated by reference to the same extent as if each reference was individually incorporated by reference. In addition, it should be understood that based on the above teaching content of the disclosure, those skilled in the art can practice various changes or modifications to the disclosure, and these equivalent forms also fall within the scope of the appended claims.

## Claims

1. A method of catalyzing a dihydrochalcone compound or a 2-hydroxyflavanone compound to generate a carboglycoside dihydrochalcone compound or a carboglycoside-2-hydroxyflavanone compound, comprising: performing the catalysis with a glycosyltransferase; the glycosyltransferase is selected from the polypeptides of any one of SEQ ID NO: 1-20 or a conservative variant thereof.

2. The method of claim 1, wherein the dihydrochalcone compound has a parent structure of formula (I), and the 2-hydroxyflavanone compound has a parent structure of formula (II), the carboglycoside dihydrochalcone compound has a parent structure of formula (III), or the carboglycoside-2-hydroxyflavanone compound has a parent structure of formula (IV): 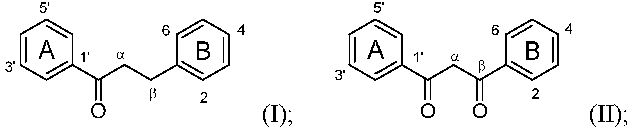 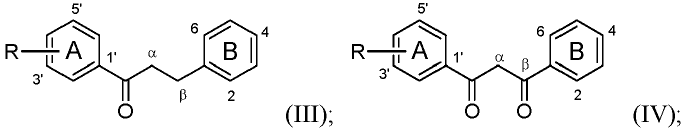 Wherein, R is a glycosyl connected with the A ring by a carbon-carbon bond.

3. The method according to claim 2, wherein, the A ring or the B ring contains 1 to 3 hydroxyl group(s); preferably, in the A ring, the hydroxyl group(s) is at position 2', 4' and/or 6'; preferably, in the B ring, the hydroxyl group(s) is at position 4.

4. The method of claim 3, wherein the number of R on the A ring is 1, preferably, R is at position 3'; or
the number of R on the A ring is 2, preferably, R is at position 3' and 5'; and the glycosyltransferase is selected from the polypeptide of SEQ ID NO: 6, 8, 10, 17, 19 or 20 or a conservative variant thereof.

5. The method of claim 4, wherein the dihydrochalcone compound or the 2-hydroxyflavanone compound comprises: phloretin, 2-hydroxynaringenin, 2-hydroxyeriodictyol; and/or
the carboglycoside dihydrochalcone compound or carboglycoside-2-hydroxyflavanone compound includes: Nothofagin, phloretin-3',5'-*C*-glucose dicarboglycoside, 2-hydroxynaringenin-6-C(8-C)-glucoside, 2-hydroxyeriodictyol-6-C(8-C)-glucoside.

6. The method of claim 2, wherein the glycosyl is glucose; preferably, when the glycosyltransferase is used for glycosyl transfer, a compound carrying a glucose group is used as a donor; more preferably, UDP glucose is used as the glycosyl donor.

7. Use of a glycosyltransferase in catalyzing a dihydrochalcone compound or a 2-hydroxyflavanone compound to generate a carboglycoside dihydrochalcone compound or a carboglycoside-2-hydroxyflavanone compound; wherein the glycosyltransferase is selected from the polypeptides of any one of SEQ ID NO: 1-20 or a conservative variant polypeptide thereof.

8. The use of claim 7, wherein the dihydrochalcone compound has a parent structure of formula (I), and the 2-hydroxyflavanone compound has a parent structure of formula (II), the carboglycoside dihydrochalcone compound has a parent structure of formula (III), or the carboglycoside-2-hydroxyflavanone compound has a parent structure of formula (IV): 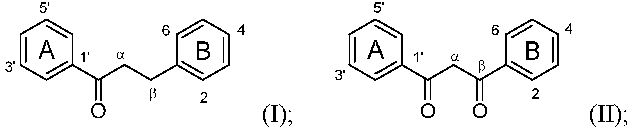 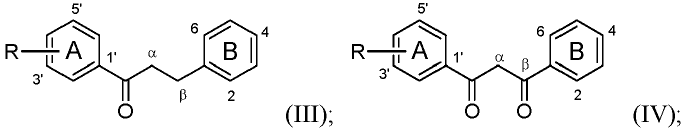 Wherein, R is a glycosyl connected with the A ring by a carbon-carbon bond.

9. The use according to claim 8, wherein, the A ring or the B ring contains 1 to 3 hydroxyl group(s); preferably, in the A ring, the hydroxyl group(s) is at position 2', 4' and/or 6'; preferably, in the B ring, the hydroxyl group(s) is at position 4.

10. The method of claim 9, wherein the number of R on the A ring is 1, preferably, R is at position 3'; or
the number of R on the A ring is 2, preferably, R is at position 3' and 5'; and the glycosyltransferase is selected from the polypeptide of SEQ ID NO: 6, 8, 10, 17, 19 or 20 or a conservative variant polypeptide thereof.

11. The use of claim 10, wherein the dihydrochalcone compound or the 2-hydroxyflavanone compound comprises: phloretin, 2-hydroxynaringenin, 2-hydroxyeriodictyol; and/or
the carboglycoside dihydrochalcone compound or carboglycoside-2-hydroxyflavanone compound includes: Nothofagin, phloretin-3',5'-*C*-glucose dicarboglycoside, 2-hydroxynaringenin-6-C(8-C)-glucoside, 2-hydroxyeriodictyol-6-C(8-C)-glucoside.

12. The method or use according to any of claims 1-11, wherein the conservative variant polypeptide comprises:
(1) a polypeptide having one or more amino acids deleted, substituted, or inserted in the sequence of any of SEQ ID NOs: 1-20, and still having the function of catalyzing a dihydrochalcone compound or a 2-hydroxyflavanone compound to generate a carboglycoside dihydrochalcone compound or a carboglycoside-2-hydroxyflavanone compound;
(2) a polypeptide having more than 80% identity with the amino acid sequence of any of SEQ ID NOs: 1-20, and still having the function of catalyzing a dihydrochalcone compound or a 2-hydroxyflavanone compound to generate a carboglycoside dihydrochalcone compound or a carboglycoside-2-hydroxyflavanone compound; or
(3) a polypeptide having a tag sequence at the N- or C-terminus of the polypeptide of any of SEQ ID NOs: 1 to 20, or having a signal peptide at its N-terminus.

13. A method of synthesizing a C-glycosylated flavone compound, comprising:
(1) catalyzing 2-hydroxyflavanone compound by a glycosyltransferase to produce carboglycoside-2-hydroxyflavanone compound; wherein, the glycosyltransferase is selected from the polypeptides of any one of SEQ ID NO: 1-20 or a conservative variant thereof;
(2) dehydrating the carboglycoside-2-hydroxyflavanone compound of (1) to obtain a C-glycosylated flavone compound.

14. The method of claim 13, further comprising step (c) before (1): (c) catalyzing the flavanone compound by flavanone-2-hydroxylase to obtain 2-hydroxyflavanone compound.

15. The method of claim 14, further comprising step (b) before (c): (b) catalyzing malonyl-CoA structural analogs and p-coumaroyl-CoA structural analogs by chalcone synthetase and chalcone isomerase to obtain flavanone compounds.

16. The method of claim 15, further comprising step (a) before (b): (a) catalyzing aromatic amino acids by tyrosine ammonia lyases or phenylalanine ammonia lyase and 4-coumaroyl-CoA ligase, to obtain p-coumaroyl-CoA structural analogs.

17. The method according to any of claims 14 to 16, wherein the flavanone compounds comprise: naringenin, eriodictyol;
the malonyl-CoA structural analogs include: malonyl-CoA or methylmalonyl-CoA;
the *p*-coumaroyl-CoA structural analogs include: *p*-coumaroyl-CoA or *p*-cinnamoyl-CoA;
the aromatic amino acids include: L-tyrosine or L-phenylalanine.

18. The method according to any of claims 13 to 16, wherein the 2-hydroxyflavanone compound is 2-hydroxynaringenin, which is obtained from naringenin catalyzed by flavanone-2-hydroxylase; or
the 2-hydroxyflavanone compound is 2-hydroxyeriodictyol, which is obtained from eriodictyol catalyzed by flavanone-2-hydroxylase; preferably, the eriodictyol is obtained from naringenin catalyzed by flavanone-3'-hydroxylase.

19. A method of biosynthesizing a C-glycosylated flavone compound, comprising:
(i) co-transforming into a host cell precursor gene(s) for the synthesis of naringenin compound, the gene encoding flavanone-2-hydroxylase and/or flavanone-3'-hydroxylase, and the gene encoding glycosyltransferase; wherein, the glycosyltransferase is selected from the polypeptides of any one of SEQ ID NO: 1-20 or a conservative variant thereof;
(ii) culturing the cells of (i) to biosynthesize C-glycosylated flavone compounds.

20. The method of claim 19, wherein the flavanone-2-hydroxylase and flavanone-3'-hydroxylase are P450 oxidase with the N-terminal transmembrane region truncated; preferably, P450 oxidase is added with a tag after truncating the N-terminal transmembrane region; more preferably, the tag includes: 2B1, 17α, MBP.

21. A genetically engineered cell, comprising precursor gene(s) for the synthesis of naringenin compound, the gene encoding flavanone-2-hydroxylase and/or flavanone-3'-hydroxylase, and the gene encoding glycosyltransferase; wherein, the glycosyltransferase is selected from the polypeptides of any one of SEQ ID NO: 1-20 or a conservative variant thereof.

22. A method for preparing the cell of claim 21, comprising: co-transforming into a host cell precursor gene(s) for the synthesis of naringenin compound, the gene encoding flavanone-2-hydroxylase and/or flavanone-3'-hydroxylase, and the gene encoding glycosyltransferase; wherein, the glycosyltransferase is selected from the polypeptides of any one of SEQ ID NO: 1-20 or a conservative variant thereof.

23. A kit for biosynthesizing a C-glycosylated flavone compound or its intermediate, comprising: one or more polypeptides shown in SEQ ID NOs: 1-20 or conservative variant polypeptides thereof; flavanone-2-hydroxylase and/or flavanone-3'-hydroxylase; precursor gene(s) for synthesizing naringenin compounds; preferably, the kit also comprising host cells; or
wherein, the kit includes the genetically engineered cell of claim 21.

24. According to any one of claims 19 to 23, the cells include: prokaryotic cells or eukaryotic cells; preferably, the prokaryotic host cells include *Escherichia coli,* yeast, streptomyces.

25. According to any of claims 13 to 23, wherein the C-glycosylated flavone compound comprises: vitexin, isovitexin, orientin, isoorientin, vitzenin-2, lucenin.
